# EUROPEAN PATENT SPECIFICATION

(11) **EP 1 948 830 B1**
(45) Date of publication and mention of the grant of the patent: **27.04.2011**
(21) Application number: 06842364.9
(22) Date of filing: 13.10.2006
(51) Int. Cl.: C12Q 1/68

(54) **GENETIC VARIATIONS ASSOCIATED WITH PSYCHIATRIC DISORDERS**
GENETISCHE VARIATIONEN IN ZUSAMMENHANG MIT PSYCHIATRISCHEN STÖRUNGEN
VARIATIONS GENETIQUES ASSOCIEES A DES TROUBLES PSYCHIATRIQUES

(30) Priority: 14.10.2005 CA 2523399
(43) Date of publication of application: 30.07.2008
(73) Proprietor: INSTITUT PASTEUR, 75724 Paris Cédex 15 (FR)
(72) Inventor: BOURGERON, Thomas, F-75013 Paris (FR); MELKE, Jonas, 41322 GOTEBORG (SE); GOUBRAN BOTROS, Hany, F-93310 Le Pre Saint Gervais (FR); LAUNAY, Jean-Marie, F-95100 Argenteuil (FR); LEBOYER, Marion, F-75016 Paris (FR); GILLBERG, Christopher, S-71319 Goteborg (SE); CHASTE, Pauline, F-75020 Paris (FR); DELORME, Richard, F-75011 Paris (FR)
(74) Representative: Orès, Bernard
(86) International application number: PCT/IB2006/003935
(87) International publication number: WO 2007/052166

(56) References cited:
- WO-A-02/076452
- US-A1- 2005 118 588
- TORDJMAN S ET AL: "Nocturnal excretion of 6-sulphatoxymelatonin in children and adolescents with autistic disorder" BIOLOGICAL PSYCHIATRY, ELSEVIER SCIENCE, NEW YORK, NY, US, vol. 57, no. 2, 15 January 2005 (2005-01-15), pages 134-138, XP004713302 ISSN: 0006-3223
- DATABASE EMBL [Online] Encoding protein CAI41504.1 (UniProt Q5JQ71) 6 March 2002 (2002-03-06), "ASMT" XP002436001 retrieved from EBI Database accession no. AL683807
- DATABASE EMBL [Online] Encoding protein AAV74311.2 (UniProt Q51S55). 1 January 2005 (2005-01-01), "AANAT" XP002436002 Database accession no. AY665273
- DATABASE EMBL [Online] Encoding protein AAB17722.1 (UniProt P49217). 22 December 1994 (1994-12-22), "Melatonin receptor Mel-1a." XP002436003 Database accession no. U14110
- BERNARD M ET AL: "Transcriptional regulation of the chicken hydroxyindole-O-methyltrans ferase gene by the cone-rod homeobox-containing protein." JOURNAL OF NEUROCHEMISTRY OCT 2001, vol. 79, no. 2, October 2001 (2001-10), pages 248-257, XP002435991 ISSN: 0022-3042
- BALER R ET AL: "The rat arylalkylamine N-acetyltransferase gene promoter. cAMP activation via a cAMP-responsive element-CCAAT complex." THE JOURNAL OF BIOLOGICAL CHEMISTRY 14 MAR 1997, vol. 272, no. 11, 14 March 1997 (1997-03-14), pages 6979-6985, XP002435992 ISSN: 0021-9258
- MUHLE R ET AL: "The genetics of autism" PEDIATRICS, AMERICAN ACADEMY OF PEDIATRICS, EVANSTON, IL,, US, vol. 113, no. 5, May 2004 (2004-05), pages e472-e486, XP002388697 ISSN: 0031-4005
- GIANNOTTI F ET AL: "LONG-TERM MELATONIN TREATMENT FOR SLEEP DISORDERS IN AUTISTIC CHILDREN. A TWO-YEAR FOLLOW-UP STUDY" SLEEP, ALLEN PRESS, LAWRENCE, KS, US, vol. 27, no. SUPPL 5, 2004, page 94, XP008057499 ISSN: 0161-8105
- PACCHIEROTTI C ET AL: "Melatonin in psychiatric disorders: a review on the melatonin involvement in psychiatry." FRONTIERS IN NEUROENDOCRINOLOGY JAN 2001, vol. 22, no. 1, January 2001 (2001-01), pages 18-32, XP002435995 ISSN: 0091-3022 cited in the application
- DOHERTY MICHAEL J ET AL: "An Xp; Yq translocation causing a novel contiguous gene syndrome in brothers with generalized epilepsy, ichthyosis, and attention deficits." EPILEPSIA, vol. 44, no. 12, December 2003 (2003-12), pages 1529-1535, XP002435996 ISSN: 0013-9580
- MELKE J ET AL.: "Abnormal melatonin synthesis in autism spectrum disorders." MOLECULAR PSYCHIATRY, 15 May 2007 (2007-05-15), pages 1-9, XP002435997

### SEQUENCE LISTING

<110> INSTITUT PASTEUR
   BOURGERON, Thomas
   MELKE, Jonas
   GOUBRAN BOTROS, Hany
   LAUNAY, Jean-Marie
   LEBOYER, Marion
   GILLBERG, Christopher
   CHASTE, Pauline
   DELORME, Richard
<120> GENETIC VARIATIONS ASSOCIATED WITH PSYCHIATRIC DISORDERS
<130> BLOcp226-134PCT
<150> CA 2,523,399
   <151> 2005-10-14
<160> 36
<170> PatentIn version 3.3
<210> 1
   <211> 373
   <212> PRT
   <213> Homo sapiens
<400> 1
   Met Gly Ser Ser Glu Asp Gln Ala Tyr Arg Leu Leu Asn Asp Tyr Ala
   1               5                   10                  15
   Asn Gly Phe Met Val Ser Gln Val Leu Phe Ala Ala Cys Glu Leu Gly
               20                  25                  30
   Val Phe Asp Leu Leu Ala Glu Ala Pro Gly Pro Leu Asp Val Ala Ala
           35                  40                  45
   Val Ala Ala Gly Val Arg Ala Ser Ala His Gly Thr Glu Leu Leu Leu
       50                  55                  60
   Asp Ile Cys Val Ser Leu Lys Leu Leu Lys Val Glu Thr Arg Gly Gly
   65                  70                  75                  80
   Lys Ala Phe Tyr Arg Asn Thr Glu Leu Ser Ser Asp Tyr Leu Thr Thr
                   85                  90                  95
   Val Ser Pro Thr Ser Gln Cys Ser Met Leu Lys Tyr Met Gly Arg Thr
               100                 105                 110
   Ser Tyr Arg Cys Trp Gly His Leu Ala Asp Ala Val Arg Glu Gly Arg
           115                 120                 125
   Asn Gln Tyr Leu Glu Thr Phe Gly Val Pro Ala Glu Glu Leu Phe Thr
       130                 135                 140
   Ala Ile Tyr Arg Ser Glu Gly Glu Arg Leu Gln Phe Met Gln Ala Leu
   145                 150                 155                 160
   Gln Glu Val Trp Ser Val Asn Gly Arg Ser Val Leu Thr Ala Phe Asp
   165 170 175
   Leu Ser Val Phe Pro Leu Met Cys Asp Leu Gly Gly Thr Arg Ile Lys
   180 185 190
   Leu Glu Thr Ile Ile Leu Ser Lys Leu Ser Gln Gly Gln Lys Thr Lys
   195 200 205
   His Arg Val Phe Ser Leu Ile Gly Gly Ala Gly Ala Leu Ala Lys Glu
   210 215 220
   Cys Met Ser Leu Tyr Pro Gly Cys Lys Ile Thr Val Phe Asp Ile Pro
   225 230 235 240
   Glu Val Val Trp Thr Ala Lys Gln His Phe Ser Phe Gln Glu Glu Glu
   245 250 255
   Gln Ile Asp Phe Gln Glu Gly Asp Phe Phe Lys Asp Pro Leu Pro Glu
   260 265 270
   Ala Asp Leu Tyr Ile Leu Ala Arg Val Leu His Asp Trp Ala Asp Gly
   275 280 285
   Lys Cys Ser His Leu Leu Glu Arg Ile Tyr His Thr Cys Lys Pro Gly
   290 295 300
   Gly Gly Ile Leu Val Ile Glu Ser Leu Leu Asp Glu Asp Arg Arg Gly
   305 310 315 320
   Pro Leu Leu Thr Gln Leu Tyr Ser Leu Asn Met Leu Val Gln Thr Glu
   325 330 335
   Gly Gln Glu Arg Thr Pro Thr His Tyr His Met Leu Leu Ser Ser Ala
   340 345 350
   Gly Phe Arg Asp Phe Gln Phe Lys Lys Thr Gly Ala Ile Tyr Asp Ala
   355 360 365
   Ile Leu Ala Arg Lys
   370
<210> 2
   <211> 345
   <212> PRT
   <213> Homo sapiens
<400> 2
   Met Gly Ser Ser Glu Asp Gln Ala Tyr Arg Leu Leu Asn Asp Tyr Ala
   1               5                   10                  15
   Asn Gly Phe Met Val Ser Gln Val Leu Phe Ala Ala Cys Glu Leu Gly
               20                  25                  30
   Val Phe Asp Leu Leu Ala Glu Ala Pro Gly Pro Leu Asp Val Ala Ala
           35                  40                  45
   Val Ala Ala Gly Val Arg Ala Ser Ala His Gly Thr Glu Leu Leu Leu
       50                  55                  60
   Asp Ile Cys Val Ser Leu Lys Leu Leu Lys Val Glu Thr Arg Gly Gly
   65                  70                  75                  80
   Lys Ala Phe Tyr Arg Asn Thr Glu Leu Ser Ser Asp Tyr Leu Thr Thr
                   85                  90                  95
   Val Ser Pro Thr Ser Gln Cys Ser Met Leu Lys Tyr Met Gly Arg Thr
               100                 105                 110
   Ser Tyr Arg Cys Trp Gly His Leu Ala Asp Ala Val Arg Glu Gly Arg
           115                 120                 125
   Asn Gln Tyr Leu Glu Thr Phe Gly Val Pro Ala Glu Glu Leu Phe Thr
       130                 135                 140
   Ala Ile Tyr Arg Ser Glu Gly Glu Arg Leu Gln Phe Met Gln Ala Leu
   145                 150                 155                 160
   Gln Glu Val Trp Ser Val Asn Gly Arg Ser Val Leu Thr Ala Phe Asp
                   165                 170                 175
   Leu Ser Val Phe Pro Leu Met Cys Asp Leu Gly Gly Gly Ala Gly Ala
               180                 185                 190
   Leu Ala Lys Glu Cys Met Ser Leu Tyr Pro Gly Cys Lys Ile Thr Val
           195                 200                 205
   Phe Asp Ile Pro Glu Val Val Trp Thr Ala Lys Gln His Phe Ser Phe
       210                 215                 220
   Gln Glu Glu Glu Gln Ile Asp Phe Gln Glu Gly Asp Phe Phe Lys Asp
   225                 230                 235                 240
   Pro Leu Pro Glu Ala Asp Leu Tyr Ile Leu Ala Arg Val Leu His Asp
                   245                 250                 255
   Trp Ala Asp Gly Lys Cys Ser His Leu Leu Glu Arg Ile Tyr His Thr
               260                 265                 270
   Cys Lys Pro Gly Gly Gly Ile Leu Val Ile Glu Ser Leu Leu Asp Glu
           275                 280                 285
   Asp Arg Arg Gly Pro Leu Leu Thr Gln Leu Tyr Ser Leu Asn Met Leu
   290 295 300
   Val Gln Thr Glu Gly Gln Glu Arg Thr Pro Thr His Tyr His Met Leu
   305 310 315 320
   Leu Ser Ser Ala Gly Phe Arg Asp Phe Gln Phe Lys Lys Thr Gly Ala
   325 330 335
   Ile Tyr Asp Ala Ile Leu Ala Arg Lys
   340 345
<210> 3
   <211> 298
   <212> PRT
   <213> Homo sapiens
<400> 3
   Met Gly Ser Ser Glu Asp Gln Ala Tyr Arg Leu Leu Asn Asp Tyr Ala
   1               5                   10                  15
   Asn Gly Phe Met Val Ser Gln Val Leu Phe Ala Ala Cys Glu Leu Gly
               20                  25                  30
   Val Phe Asp Leu Leu Ala Glu Ala Pro Gly Pro Leu Asp Val Ala Ala
           35                  40                  45
   Val Ala Ala Gly Val Arg Ala Ser Ala His Gly Thr Glu Leu Leu Leu
       50                  55                  60
   Asp Ile Cys Val Ser Leu Lys Leu Leu Lys Val Glu Thr Arg Gly Gly
   65                  70                  75                  80
   Lys Ala Phe Tyr Arg Asn Thr Glu Leu Ser Ser Asp Tyr Leu Thr Thr
                   85                  90                  95
   Val Ser Pro Thr Ser Gln Cys Ser Met Leu Lys Tyr Met Gly Arg Thr
               100                 105                 110
   Ser Tyr Arg Cys Trp Gly His Leu Ala Asp Ala Val Arg Glu Gly Arg
           115                 120                 125
   Asn Gln Tyr Leu Glu Thr Phe Gly Val Pro Ala Glu Glu Leu Phe Thr
       130                 135                 140
   Ala Ile Tyr Arg Ser Glu Gly Glu Arg Leu Gln Phe Met Gln Ala Leu
   145                 150                 155                 160
   Gln Glu Val Trp Ser Val Asn Gly Arg Ser Val Leu Thr Ala Phe Asp
                   165                 170                 175
   Leu Ser Val Phe Pro Leu Met Cys Asp Leu Gly Gly Asp Phe Phe Lys
   180 185 190
   Asp Pro Leu Pro Glu Ala Asp Leu Tyr Ile Leu Ala Arg Val Leu His
   195 200 205
   Asp Trp Ala Asp Gly Lys Cys Ser His Leu Leu Glu Arg Ile Tyr His
   210 215 220
   Thr Cys Lys Pro Gly Gly Gly Ile Leu Val Ile Glu Ser Leu Leu Asp
   225 230 235 240
   Glu Asp Arg Arg Gly Pro Leu Leu Thr Gln Leu Tyr Ser Leu Asn Met
   245 250 255
   Leu Val Gln Thr Glu Gly Gln Glu Arg Thr Pro Thr His Tyr His Met
   260 265 270
   Leu Leu Ser Ser Ala Gly Phe Arg Asp Phe Gln Phe Lys Lys Thr Gly
   275 280 285
   Ala Ile Tyr Asp Ala Ile Leu Ala Arg Lys
   290 295
<210> 4
   <211> 207
   <212> PRT
   <213> Homo sapiens
<400> 4
   Met Ser Thr Gln Ser Thr His Pro Leu Lys Pro Glu Ala Pro Arg Leu
   1 5 10 15
   Pro Pro Gly Ile Pro Glu Ser Pro Ser Cys Gln Arg Arg His Thr Leu
   20 25 30
   Pro Ala Ser Glu Phe Arg Cys Leu Thr Pro Glu Asp Ala Val Ser Ala
   35 40 45
   Phe Glu Ile Glu Arg Glu Ala Phe Ile Ser Val Leu Gly Val Cys Pro
   50 55 60
   Leu Tyr Leu Asp Glu Ile Arg His Phe Leu Thr Leu Cys Pro Glu Leu
   65 70 75 80
   Ser Leu Gly Trp Phe Glu Glu Gly Cys Leu Val Ala Phe Ile Ile Gly
   85 90 95
   Ser Leu Trp Asp Lys Glu Arg Leu Met Gln Glu Ser Leu Thr Leu His
   100 105 110
   Arg Ser Gly Gly His Ile Ala His Leu His Val Leu Ala Val His Arg
   115 120 125
   Ala Phe Arg Gln Gln Gly Arg Gly Pro Ile Leu Leu Trp Arg Tyr Leu
   130 135 140
   His His Leu Gly Ser Gln Pro Ala Val Arg Arg Ala Ala Leu Met Cys
   145 150 155 160
   Glu Asp Ala Leu Val Pro Phe Tyr Glu Arg Phe Ser Phe His Ala Val
   165 170 175
   Gly Pro Cys Ala Ile Thr Val Gly Ser Leu Thr Phe Met Glu Leu His
   180 185 190
   Cys Ser Leu Arg Gly His Pro Phe Leu Arg Arg Asn Ser Gly Cys
   195 200 205
<210> 5
   <211> 350
   <212> PRT
   <213> Homo sapiens
<400> 5
<210> 6
   <211> 362
   <212> PRT
   <213> Homo sapiens
<400> 6
   Met Ser Glu Asn Gly Ser Phe Ala Asn Cys Cys Glu Ala Gly Gly Trp
   1               5                   10                  15
   Ala Val Arg Pro Gly Trp Ser Gly Ala Gly Ser Ala Arg Pro Ser Arg
               20                  25                  30
   Thr Pro Arg Pro Pro Trp Val Ala Pro Ala Leu Ser Ala Val Leu Ile
           35                  40                  45
   Val Thr Thr Ala Val Asp Val Val Gly Asn Leu Leu Val Ile Leu Ser
       50                  55                      60
   Val Leu Arg Asn Arg Lys Leu Arg Asn Ala Gly Asn Leu Phe Leu Val
   65                  70                  75                  80
   Ser Leu Ala Leu Ala Asp Leu Val Val Ala Phe Tyr Pro Tyr Pro Leu
                   85                  90                  95
   Ile Leu Val Ala Ile Phe Tyr Asp Gly Trp Ala Leu Gly Glu Glu His
               100                 105                 110
   Cys Lys Ala Ser Ala Phe Val Met Gly Leu Ser Val Ile Gly Ser Val
           115                 120                 125
   Phe Asn Ile Thr Ala Ile Ala Ile Asn Arg Tyr Cys Tyr Ile Cys His
       130                 135                 140
   Ser Met Ala Tyr His Arg Ile Tyr Arg Arg Trp His Thr Pro Leu His
   145                 150                 155                 160
   Ile Cys Leu Ile Trp Leu Leu Thr Val Val Ala Leu Leu Pro Asn Phe
                   165                 170                 175
   Phe Val Gly Ser Leu Glu Tyr Asp Pro Arg Ile Tyr Ser Cys Thr Phe
               180                 185                 190
   Ile Gln Thr Ala Ser Thr Gln Tyr Thr Ala Ala Val Val Val Ile His
           195                 200                 205
   Phe Leu Leu Pro Ile Ala Val Val Ser Phe Cys Tyr Leu Arg Ile Trp
       210                 215                 220
   Val Leu Val Leu Gln Ala Arg Arg Lys Ala Lys Pro Glu Ser Arg Leu
   225                 230                 235                 240
   Cys Leu Lys Pro Ser Asp Leu Arg Ser Phe Leu Thr Met Phe Val Val
                   245                 250                 255
   Phe Val Ile Phe Ala Ile Cys Trp Ala Pro Leu Asn Cys Ile Gly Leu
               260                 265                 270
   Ala Val Ala Ile Asn Pro Gln Glu Met Ala Pro Gln Ile Pro Glu Gly
           275                 280                 285
   Leu Phe Val Thr Ser Tyr Leu Leu Ala Tyr Phe Asn Ser Cys Leu Asn
       290                 295                 300
   Ala Ile Val Tyr Gly Leu Leu Asn Gln Asn Phe Arg Arg Glu Tyr Lys
   305                 310                 315                 320
   Arg Ile Leu Leu Ala Leu Trp Asn Pro Arg His Cys Ile Gln Asp Ala
   325 330 335
   Ser Lys Gly Ser His Ala Glu Gly Leu Gln Ser Pro Ala Pro Pro Ile
   340 345 350
   Ile Gly Val Gln His Gln Ala Asp Ala Leu
   355 360
<210> 7
   <211> 21
   <212> DNA
   <213> Artificial
<220>
   <223> PCR primer
<400> 7
   gagcgattct tctgcctcag c 21
<210> 8
   <211> 20
   <212> DNA
   <213> Artificial
<220>
   <223> PCR primer
<400> 8
   tctgcgcaca ctcccaggtg 20
<210> 9
   <211> 21
   <212> DNA
   <213> Artificial
<220>
   <223> PCR primer
<400> 9
   gaggcaggag aatcgcttga a 21
<210> 10
   <211> 22
   <212> DNA
   <213> Artificial
<220>
   <223> PCR primer
<400> 10
   ggctacatcg tgggtgtacg tc 22
<210> 11
   <211> 23
   <212> DNA
   <213> Artificial
<220>
   <223> PCR primer
<400> 11
   tggtgcaatc tcatttgact ctg 23
<210> 12
   <211> 20
   <212> DNA
   <213> Artificial
<220>
   <223> PCR primer
<400> 12
   gggttcatgc cattctcctg 20
<210> 13
   <211> 21
   <212> DNA
   <213> Artificial
<220>
   <223> PCR primer
<400> 13
   cagctgtaca aggcaagagg a 21
<210> 14
   <211> 21
   <212> DNA
   <213> Artificial
<220>
   <223> PCR primer
<400> 14
   ctttcacctc ctccactgcc a 21
<210> 15
   <211> 21
   <212> DNA
   <213> Artificial
<220>
   <223> PCR primer
<400> 15
   gcctgggcta cagagctgaa a 21
<210> 16
   <211> 20
   <212> DNA
   <213> Artificial
<220>
   <223> PCR primer
<400> 16
   ctcctgggtt gtgccatttg 20
<210> 17
   <211> 22
   <212> DNA
   <213> Artificial
<220>
   <223> PCR primer
<400> 17
   cctgtggggt atagctccgt tc 22
<210> 18
   <211> 21
   <212> DNA
   <213> Artificial
<220>
   <223> PCR primer
<400> 18
   cgcacatgtc aaagcatcag a 21
<210> 19
   <211> 21
   <212> DNA
   <213> Artificial
<220>
   <223> PCR primer
<400> 19
   agcttgcagt gagcggaaat c 21
<210> 20
   <211> 21
   <212> DNA
   <213> Artificial
<220>
   <223> PCR primer
<400> 20
   gcacccatcg actcgtcatt t 21
<210> 21
   <211> 21
   <212> DNA
   <213> Artificial
<220>
   <223> PCR primer
<400> 21
   tgggttggac ccttcatgag t 21
<210> 22
   <211> 21
   <212> DNA
   <213> Artificial
<220>
   <223> PCR primer
<400> 22
   gtgtttccgg gagtgagagg a 21
<210> 23
   <211> 21
   <212> DNA
   <213> Artificial
<220>
   <223> PCR primer
<400> 23
   agcctggaag acctgggaaa g 21
<210> 24
   <211> 21
   <212> DNA
   <213> Artificial
<220>
   <223> PCR primer
<400> 24
   cctgtgggat gatttcagtg c 21
<210> 25
   <211> 21
   <212> DNA
   <213> Artificial
<220>
   <223> PCR primer
<400> 25
   ggtgccctga ctgtcctctg a 21
<210> 26
   <211> 21
   <212> DNA
   <213> Artificial
<220>
   <223> PCR primer
<400> 26
   ccatcagcgt ggtcctcagt a 21
<210> 27
   <211> 430
   <212> DNA
   <213> Homo sapiens
<400> 27
   caggacctgc tcaatccata agacgattaa tgagtgctaa tgatgggtga gcatattggt 60
   ttgggatcag ttctacagct gatgagtcaa gggctcgggg gggcctgatg cagactattt 120
   tagggctgcg gaggggcgcc cagaaagcca gggaatcgcc acacgctttg tgatccccta 180
   atacggttaa tgcaaatttc tagagtgatc cgtctttgtt tgagtactgg gcaggcagca 240
   gggagagtca ggcagcagct gtgagcgggt ggctcttccc caccttgcca gcaggctctg 300
   tgctccttga agcaagcgct ccagaggctc cggaagccac ggctggattg gagacaagat 360
   gggatcctca gaggaccagg cctatcgcct ccttaatgac tacgccaacg gcttcatggt 420
   gtcccaggta 430
<210> 28
   <211> 23
   <212> PRT
   <213> Homo sapiens
<400> 28
   Met Gly Ser Ser Glu Asp Gln Ala Tyr Arg Leu Leu Asn Asp Tyr Ala
   1 5 10 15
   Asn Gly Phe Met Val Ser Gln
   20
<210> 29
   <211> 59
   <212> DNA
   <213> Homo sapiens
<220>
   <221> variation
   <222> (27)..(27)
   <223> y = t or c
<400> 29
   ttcccactta tgtgtgacct tggtggyaag tacccctcac cactaatacc tcggaggtg 59
<210> 30
   <211> 65
   <212> PRT
   <213> Homo sapiens
<400> 30
   Trp Ala Asp Gly Lys Cys Ser His Leu Leu Glu Arg Ile Tyr His Thr
   1 5 10 15
   Cys Lys Pro Gly Gly Ala Ile Leu Val Ile Glu Ser Leu Leu Asp Glu
   20 25 30
   Asp Arg Arg Gly Pro Leu Leu Thr Gln Phe Tyr Ser Leu Asn Met Leu
   35 40 45
   Val Gln Thr Glu Gly Gln Glu Arg Thr Pro Thr His Tyr His Met Leu
   50 55 60
   Leu
   65
<210> 31
   <211> 65
   <212> PRT
   <213> Macaca
<400> 31
   Trp Ala Asp Gly Lys Cys Ser His Leu Leu Glu Arg Val Tyr His Thr
   1 5 10 15
   Cys Lys Pro Gly Gly Gly Ile Leu Val Ile Glu Ser Leu Leu Asp Glu
   20 25 30
   Asp Arg Arg Gly Pro Leu Leu Thr Gln Leu Tyr Ser Leu Asn Met Leu
   35 40 45
   Val Gln Thr Glu Gly Gln Glu Arg Thr Pro Thr His Tyr His Met Leu
   50 55 60
   Leu
   65
<210> 32
   <211> 65
   <212> PRT
   <213> Bovine
<400> 32
   Trp Thr Asp Ala Lys Cys Ser His Leu Leu Gln Arg Val Tyr Arg Ala
   1 5 10 15
   Cys Arg Thr Gly Gly Gly Ile Leu Val Ile Glu Ser Leu Leu Asp Thr
   20 25 30
   Asp Gly Arg Gly Pro Leu Thr Thr Leu Leu Tyr Ser Leu Asn Met Leu
   35 40 45
   Val Gln Thr Glu Gly Arg Glu Arg Thr Pro Ala Glu Tyr Arg Ala Leu
   50 55 60
   Leu
   65
<210> 33
   <211> 65
   <212> PRT
   <213> Rattus
<400> 33
   Trp Ala Asp Gly Ala Cys Val Glu Leu Leu Gly Arg Leu His Arg Ala
   1 5 10 15
   Cys Thr Ser Ser Gly Ala Leu Leu Leu Val Glu Ala Val Leu Ala Lys
   20 25 30
   Gly Gly Ala Gly Pro Leu Arg Ser Leu Leu Leu Ser Leu Asn Met Met
   35 40 45
   Leu Gln Ala Glu Gly Trp Glu Arg Gln Ala Ser Asp Tyr Arg Asn Leu
   50 55 60
   Ala
   65
<210> 34
   <211> 65
   <212> PRT
   <213> Gallus
<400> 34
   Trp Asp Asp Lys Lys Cys Arg Gln Leu Leu Ala Glu Val Tyr Lys Ala
   1 5 10 15
   Cys Arg Pro Gly Gly Gly Val Leu Leu Val Glu Ser Leu Leu Ser Glu
   20 25 30
   Asp Arg Ser Gly Pro Val Glu Thr Gln Leu Tyr Ser Leu Asn Met Leu
   35 40 45
   Val Gln Thr Glu Gly Lys Glu Arg Thr Ala Val Glu Tyr Ser Glu Leu
   50 55 60
   Leu
   65
<210> 35
   <211> 62
   <212> PRT
   <213> Danio
<400> 35
   Trp Ser Asp Glu Lys Leu His Val Leu Leu Ser Lys Leu Cys Lys Met
   1 5 10 15
   Trp Thr Pro Gly Cys Gly Leu Leu Val Ser Glu Ile Leu Leu Asp Glu
   20 25 30
   Glu Arg Arg Arg Pro Ser Arg Ala Leu Leu Gln Ala Leu Ser Met Thr
   35 40 45
   Glu Gly Lys Gln Arg Ser Thr Ala Glu Tyr Ser Ala Leu Leu
   50 55 60
<210> 36
   <211> 65
   <212> PRT
   <213> Fugu
<400> 36
   Trp Thr Asp Gln Arg Cys Leu Glu Leu Leu Arg Arg Val His Gly Ala
   1 5 10 15
   Cys Arg Pro Gly Gly Ser Val Leu Leu Val Glu Ala Leu Leu Tyr Glu
   20 25 30
   Asp Gly Ser Gly Pro Leu Thr Ala Gln Leu Tyr Ser Leu Asn Met Leu
   35 40 45
   Val Gln Thr Glu Gly Arg Glu Arg Ser Ala Ala Gln Tyr Ala Ala Leu
   50 55 60
   Leu
   65

## Description

### FIELD OF THE INVENTION

The present invention relates to methods for determining likelihood of an individual of developing a psychiatric disorder. More particularly, the method of the invention is based on the identification of genetic variations in genes involved in the modulation of melatonin and their consequences on such pathway for the determination of whether an individual is susceptible of being afflicted by the psychiatric disorders autism, spectrum disorders and Attention Deficits and Hyper Activity Disorder (ADHD).

### BRIEF DESCRIPTION OF THE PRIOR ART

### Autism spectrum disorders (ASD) and Attention-deficit/hyperactivity disorder (ADHD)

ASD and ADHD are among the most predominant psychiatric syndromes in children and seem to be strongly influenced by genes ¹⁻⁴. ASD is characterised by impairments in communication skills, social interaction and restricted, repetitive and stereotyped patterns of behaviour ⁵. ASD includes autistic disorder (classical autism), disintegrative disorder, pervasive development disorder not otherwise specified (PDD-NOS) and Asperger syndrome. The recurrence risk of autism in sib-ships is approximately 45 times greater than in the general population and twin studies have documented a higher concordance rate in monozygotic (60%-91%) than in dizygotic twins (0%-6%) ¹. Furthermore, approximately 10% of individuals with autism have chromosomal rearrangements (e.g. duplication of 15q) or mutations in genes associated with other syndromes such as *FMR1* (Fragile X syndrome), *TSC1* and *TSC2* (tuberous sclerosis) or *NF1* (Neurofibromatosis).

ADHD is the most commonly diagnosed behavioural disorder in childhood affecting a relatively stable rate of 8-12% of all young children and likely represents an extreme of normal behaviour ⁶. It is a condition characterized by behavioural symptoms of inattention and/or hyperactivity-impulsivity, with onset in childhood ⁵. Such symptoms include restlessness, difficulty with organizing tasks, distractibility, forgetfulness, difficulty awaiting turns, and frequent interrupting. ADHD significantly impacts learning in school-age children and leads to impaired functioning throughout the life span. Family, twin, and adoption studies provided compelling evidence that genes play a strong role in mediating susceptibility to ADHD ^{3,4}.

Despite the clear clinical boundaries between these two disorders, there are behavioural, cognitive, and neurobiological deficits that suggest some degree of phenotypic overlap such as maladaptive social functioning and executive function deficits ^{7,8}. Furthermore, there is a significant subgroup of children with ASD and symptoms of ADHD who respond to stimulant medication⁹, suggesting that common neurobiological mediators may be present in a subset of cases. Shared susceptibility genes for ASD and ADHD were also suggested from the results of genetic studies, which pointed at the same chromosomal regions using linkage analyses (e.g. 16p13 and 17p11) ¹⁰ or chromosomal rearrangements (e.g. Xp22.3) ^{11,12,13}.

### ASMT (Acetyl serotonin methyl transferase) gene and AA NAT (Arylalkylamine acetyltransferase) gene.

ASMT gene is located on the pseudo autosomal region 1 (PAR1), common to the X and the Y chromosome ¹⁴ . *ASMT* encodes the enzyme HIOMT (Hydroxyindole-O-methyltransferase ; EC 2.1.1.4 ), which catalyses the last step of melatonin biosynthesis. Melatonin (N-acetyl-5-methoxytryptamine) is the major secretory product of the pineal gland. It is produced through the conversion of tryptophan, first to serotonin, then N-acetylserotonin, and finally to melatonin^{15,16}. The synthesis of melatonin exhibits a pronounced circadian rhythm: its concentration in the body is typically lower during the day and reaches a maximal level at night in the darkness ¹⁵. The regulation of melatonin production in the pineal gland involves especially norepinephrine (NE) couples to beta-adrenergic receptors ¹⁷. Melatonin has been functionally linked to the regulation of circadian and seasonal rhythms, immune function, and is a powerful free radical scavenger and antioxidant ^{15,18}. Nevertheless, the consequence of the clock and calendar information that the melatonin cycle imparts to the organism is still not fully understood.

The enzyme AA.NAT is located before ASMT in the biosynthesis pathway and transforms the serotonin into N. acetyl serotonin.

Atypical circadian rhythms are frequently observed in individuals with neuropsychiatric disorders and numerous studies have reported abnormal synthesis of melatonin or benefit from treatment with melatonin ^{19-21 22-25 26}. WO 02/076452 and WO 2004/028532 describe the use of melatonin in the treatment of ADHD. However, neither of these studies could discriminate between a cause or a consequence of the disorders.

### The melatonin receptors

In humans, two distinct melatonin receptors MTNR1A and MTNR1B have been reported so far. Both types of receptors were identified in a wide variety of tissues with different expression profiles. A third melatonin related receptor GPR50 was very recently identified. Although this receptor is structurally related to the melatonin receptors, with a 45% homology at the amino acid levels, it does not bind to melatonin. Nevertheless, the heterodimer MTNR1A/GPR50 abolishes high-affinity agonist binding and G protein coupling to the MTNR1A.

Activation of the MTNR1A leads to inhibition of forskolin stimulated cAMP formation, PKA activity, and phosphorylation of the cAMP-responsive element binding protein, a transcription factor. Activation of the MTNR1A also increases phosphorylation of mitogen-activated protein kinase and MEK1-2 and ERK1/2 probably leading to induction of synthesis of filamentous structures in non-neuronal tissues. Melatonin induction of filamentous structures in non-neuronal cells is dependent on expression of the human MTNR1A melatonin receptor. MTNR1A is coupled to parallel signal transduction pathways and regulates functional responses of melatonin in ion channels. Similar to the second messenger pathways of the MTNR1A receptor, activation of the MTNR1B also inhibits forskolin stimulated cAMP formation. Additionally coupling to this receptor can also lead to inhibition of cGMP formation.

Deletion of the large C-terminal tail of GPR50 suppresses the inhibitory effect of GPR50 on MT1 without affecting heterodimerization, indicating that this domain regulates the interaction of regulatory proteins to MT1. This effect appears to be specific for the GPR50/MT1 heterodimer since it was not observed for heterodimers with the closely related MT2. In mammals this receptor has been detected in various brain structures and peripheral tissues.

In humans, MTNR1A and MTNR1B were found in the hippocampus, throughout the cerebellar cortex in distinct cell populations. MTNR1A is localized in various areas related to dopaminergic behaviors, including Brodmann area 10 (i.e. prefrontal cortex), putamen, substantia nigra, amydala, and hippocampus. Melatonin may exert inhibitory effects, especially in the night, where melatonin levels are high.

There is thus a need for new tools marker associated with susceptibility to psychiatric disorders. Consequently, on the bases of both genetics and biochemical data, the inventors of the present invention propose that mutations in the *ASMT* gene cause an absence or a decrease of melatonin and confer an increased risk to neuropsychiatric disorders such as ASD and ADHD. The inventors thus demonstrate that some genetic variations in genes involved in melatonin biosynthesis cause an absence or a decrease of melatonin during childhood and adulthood and confer a risk to the psychiatric disorders autism and ADHD and thus provide new diagnostic methods with regards to such psychiatric disorders.

### SUMMARY OF THE INVENTION

The invention provides a method for determining the likelihood of an individual developing a psychiatric disorder selected from the group autism spectrum disorders (ASD), Attention Deficits and Hyper Activity Disorder (ADHD); comprising:
- identifying at least one genetic variation in a gene selected from the group consisting of ASMT, AANAT, involved in the modulation of melatonin and/or
- assaying the enzymatic activity of an enzyme encoded by said ASMT gene involved in the melatonin biosynthesis pathway in a sample of said individual,
said method may also comprise the step of:
- assaying the melatonin level,
whereby identification of said at least one genetic variation and/or decrease in said enzymatic activity as compared to a psychiatric-disorder free individual, is indicative of a likelihood that said individual develops a psychiatric disorder.

### BRIEF DESCRIPTION OF THE FIGURES

**Figure 1****:** Linkage and association of *ASMT* with ASD and ADHD. **a.** Linkage analysis of the PAR1 using 71 families with ASD. **b.** Linkage disequilibrium map of the ASMT gene. c. Association study of the ASMT gene in ASD and ADHD.
**Figure 2****:** Analysis of the *ASMT* mRNA in BLCL from ASD and controls. **a.** relative proportion of *ASMT* isoforms. Insert. *ASMT* isoforms from a control (C5), two ASD (A39 and A40), and cDNA from pineal gland. **b.** Relative abundance of *ASMT* correlated with rs4446909 genotype. **c.** Relative abundance of *ASMT* correlated with rs5989681 genotype. **d.** Promoter B sequence of the ASMT gene (SEQ ID NO:27 and SEQ ID NO:28). Rare and frequent variations identified in ASD are indicated in red and purple, respectively.
**Figure 3****:** Biochemical analyses in blood and BLCL from ASD, parents and controls. **a.** Serotonin concentration in the blood. **b.** ASMT activity in blood platelets. **c.** Melatonin concentration in the blood. **d.** ASMT activity in BLCL. C1 control age-matched for parents; C2 control age-matched for ASD.
**Figure 4****:** ASMT transcript level and activity in individuals with ASD and controls. ASD with GG and CG genotype at rs5989681 are in red and orange circle, respectively. Controls with GG and CG genotype at rs5989681 are in green and blue square, respectively. Individuals with low medium ASMT isoform and non-synonymous mutations are indicated by double lines and arrows, respectively.
**Figure 5****:** Position and segregation of the ASMT mutations in families with ASD, ADHD and anorexia.
**Figure 6****:** Melatonin concentration in the boy with ASD and their parents during the night. 0 is 20h30.
**Figure 7****:** Amino acid sequence of the HIOMT enzyme alternatively spliced with exon 6 and 7 (SEQ ID NO: 1; NCBI accession number AAA75290).
**Figure 8****:** Amino acid sequence of the HIOMT enzyme alternatively spliced with exon 7 and lacking exon 6 (SEQ ID NO: 2; NCBI accession number AAA75291).
**Figure 9****:** Amino acid sequence of the HIOMT enzyme lacking exon 6 and 7 (SEQ ID NO: 3; NCBI accession number AAA75289).
**Figure 10****:** Amino acid sequence of the AA.NAT enzyme (SEQ ID NO: 4; NCBI accession number NM_ 001088).
**Figure 11****: A.** Localisation of the variations within the MTNR1A/MTNR1B receptors. **B.** Family with ADHD and the STOP mutation of MTNR1A. The variations changing amino acids highly or less conserved during evolution are indicated in red and orange respectively. The amino acids, which directly bind to melatonin are indicated in blue.
**Figure 12****:** Amino acid sequence of the melatonin MTNR1A receptor (SEQ ID NO: 5; NCBI accession number P48039).
**Figure 13****:** Amino acid sequence of the melatonin MTNR1B receptor (SEQ ID NO: 6; NCBI accession number AAS00461).

### DETAILED DESCRIPTION OF THE INVENTION

### Method of diagnosis

The present invention provides a method for determining likelihood of an individual of developing autism spectrum disorders (ASD) or Attention Deficits and Hyper Activity Disorder (ADHD).

More specifically, the method of the invention is preferably achieved by identifying at least one genetic variation in a gene involved in the modulation of melatonin, wherein the susceptibly genes for psychiatric disorders contemplated by the method of the invention are ASMT (Acetyl serotonin methyl transferase) and AA-NAT (Arylalkylamine N.acetyl transferase) genes.

As used herein, the expression "genetic variation of a gene" refers to any variation that may occur within the DNA sequence of the genes contemplated by the present invention. Such a DNA sequence comprises, but is not limited to, regulatory sequences such as promoters, introns, exons, and coding sequences. By the term "genetic variation", it is meant any variation that could interfere with the transcription and/or translation of a specific gene. Genetic variation may be recombination events or mutations such as substitution/deletion/insertion events like point and splice site mutations.

Therefore, the invention relates to a method for determining the likelihood of an individual developing a psychiatric disorder selected from the group autism spectrum disorders (ASD), Attention Deficits and Hyper Activity Disorder (ADHD); comprising:
- identifying at least one genetic variation in a gene selected from the group consisting of ASMT, AANAT, involved in the modulation of melatonin and/or
- assaying the enzymatic activity of an enzyme encoded by said ASMT gene involved in the melatonin biosynthesis pathway in a sample of said individual,
said method may also comprise the step of:
- assaying the melatonin level,
whereby identification of said at least one genetic variation and/or decrease in said enzymatic activity as compared to a psychiatric-disorder free individual, is indicative of a likelihood that said individual develops a psychiatric disorder.

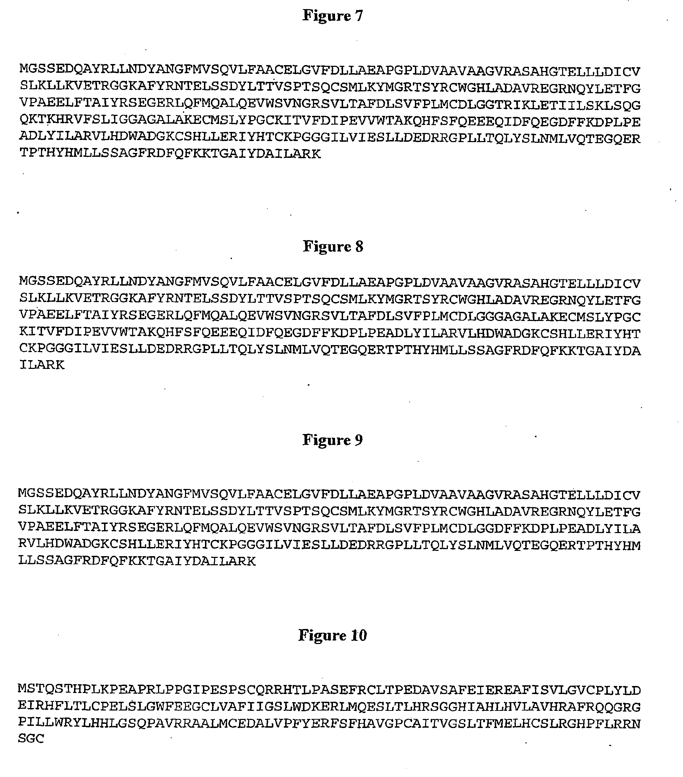

With respect to the ASMT gene, preferred genetic variations that the method of the invention is interested in, are the following preferred point mutations which give rise to a mutation located at position 17, 81, 210, 306 or 326 as defined by the position in SEQ ID NO:1 (figure 7). More preferably the mutation in SEQ ID NO:1 is selected from the group consisting of a N17K mutation, a K81E mutation, a R210H mutation, a G306A mutation and a L326F mutation. According to other preferred genetic variations regarding the ASMT gene concern point mutations which give rise to a mutation located at position 17, 81 or 298 as defined by the position in SEQ ID NO:2 (figure 8) or 17, 81, 231 or 251 as defined by the position in SEQ ID NO:3 (figure 9).

Another preferred genetic variation regarding the ASMT gene is a splice site mutation consisting of IVS5+2T>C.

Another preferred genetic variation rearding the ASMT gene is a mutation in a single nucleotide polymorphism (SNP) such as Rs5989681 or Rs6588809.

With respect to the AA. NAT gene, preferred genetic variations that the method of the invention is interested in, are the following preferred point mutations which give rise to a mutation located at position 3, 13, 62, 157 or 163 as defined by the position in SEQ ID NO:4 (figure 10). More preferably the mutation in SEQ ID NO:4 is selected from the group consisting of a T3M mutation, a A13S mutation, a V621 mutation, a A157V mutation and a A163V mutation.

According to another preferred embodiment, the method of the invention can be achieved by assaying in a sample of the individual the enzymatic activity of HIOMT (Hydroxyindole-O-methyltransferase), involved in the melatonin biosynthesis pathway. It will be understood that any suitable assay method known to one skilled in the art, such as the one described in Chanut et al.²⁸ and Finocchiaro et al.²⁹, is within the scope of the present invention.

According to another preferred embodiment, the method of the invention may also comprise the step of assaying in a sample of the individual the melatonin level. Methods for assaying melatonin levels are known by one skilled in the art (see Tordjman *et al.*²⁵, Chanut *et al.*²⁸ and Finocchiaro *et al*.²⁹).

As used herein, the term "sample" refers to a variety of sample types obtained from an individual and can be used in accordance with the method of the invention. The definition encompasses blood, saliva, urine and other samples of biological origin.

As one skilled in that art may appreciate in view of the above, identification of a genetic variation and/or decrease in the enzymatic activity of said enzyme as compared to a psychiatric-free individual is indicative of a higher likelihood that the individual develops ASD or ADHD.

The present invention will be more readily understood by referring to the following example. This example is illustrative of the wide range of applicability of the present invention. Although any methods and materials similar or equivalent to those described herein can be used in the practice for testing of the present invention, the preferred methods and materials are described.

### EXAMPLE 1

### Mutations in the ASMT and AA-NAT gene decrease melatonin synthesis and confer susceptibility to psychiatric disorders.

During the search of susceptibility factors to autism spectrum disorders (ASD) or Attention Deficits and Hyper Activity Disorder (ADHD), the inventors identified the ASMT (Acetyl serotonin methyl transferase) gene and the AA.NAT gene as a susceptibility gene for neuropsychiatric disorders. This gene is located on the pseudo autosomal region 1 (PAR1), common to the X and the Y chromosome ¹⁴. ASMT encodes the enzyme HIOMT (Hydroxyindole-O-methyltransferase ; EC 2.1.1.4), which catalyses the last step of melatonin biosynthesis ^{15,16}.

Polymorphisms located in the promoter or the coding sequence and rare mutations affecting the splicing and the protein function are associated with a decrease of ASMT transcript level and/or activity. In individuals carrying these genetic variations, the decrease in ASMT activity leads to a decrease in melatonin concentration. Melatonin (N-acetyl-5-methoxytryptamine) is the major secretory product of the pineal gland. The synthesis of melatonin exhibits a pronounced circadian rhythm: its concentrations in the body are typically lower during the day and reach maximal levels at night in the darkness.

### MATERIALS AND METHODS

### Mutation screening and genotyping

Both genotyping and screening for mutation was performed by direct sequencing. Coding regions including the exon/intron boundaries, the 5' and 3' untranslated regions, and the regulatory regions of the *ASMT* gene were amplified by PCR using HotstarTaq (Qiagen). For primers and PCR conditions, see table 8. Sequencing of PCR products was performed using the BigDye Terminator Cycle Sequencing Kit (V3.1, Applied Biosystems). Samples were then subjected to electrophoresis using an ABI PRISM genetic analyzer (Applied Biosystems). ABI electropherogram data were imported and analyzed for variation using the Genalys software (Author: Masazumi Takahashi).

### RT-PCR and Quantitative RT-PCR

For analysis of *ASMT* transcripts from the investigated subjects, RNA was isolated from the generated lymphoblastoid cell lines using the NucleoSpin® RNA II kit (MACHEREY-NAGEL). Quantification of total RNA was performed by measurement of absorbance at OD₂₆₀ on a Biophotometer (Eppendorf). Oligo(dT) primed cDNA was prepared from 5µg of this RNA using superscript II (Invitrogen) according to the manufacturers instructions in a reaction volume of 40 µl. For control of DNA contamination, a parallel tube without reverse transcriptase (RT negative control) was included in the RT reactions. All cDNA samples were tested on an agarose gel (3%) using a GAPDH PCR (for primers, see table 8), followed by a dilution with 40 to 80 µl dH2O (DEPC) in order to homogenize samples for real-time PCR analysis. The cDNA was used directly in TaqMan assays using the ABI PRISM 7500 Sequence Detection System (PE Biosystems). Each reaction was performed in triplicate and the cDNA was added to each reaction (25µL) containing 1.25 µL of the assay and 12.5 µL of the TaqMan Universal PCR Master Mix (Applied Biosystems). Study samples were run in duplicate or triplicate on 96-well optical PCR plates (ABgene). Quantification of *ASMT* mRNA was performed using commercially available Assays-on-Demand. Two different assays were used, one covering the boundary between exon 1B and exon 2 (Hs00946625_m1), and one covering the boundary between exon 8 and exon 9 (Hs00187839_m1). Relative values of expression were determined for each sample using the standard curve method (ABI user's manual), and these values were normalized to the Ct values of GAPDH, a standard "housekeeping" control gene, using the glyceraldehyde phosphate dehydrogenase assay Hs99999905_m1. For *ASMT* and *GAPDH,* the thresholds were set at 0.2 and 0.25, respectively, which was within the linear region of the semi-log plot in all assays.

### RESULTS

### Linkage between the pseudo autosomal region 1 and ASD

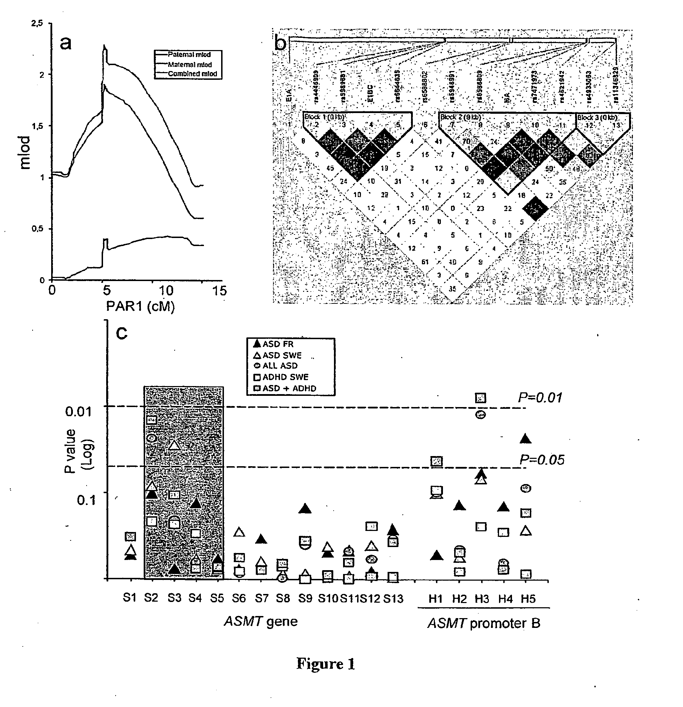

The Pseudo Autosomal Region 1 (PAR1) is only 2.7 Mb of DNA located on the tip of the short arms of the sex chromosomes and containing fifteen genes. A fine mapping of the PAR1 using four microsatellites markers (DXYS233, DXYS234, DXYS228 and DXYS229) was conducted on 52 families with at least two children with ASD. The inventors observed an excess of allelic sharing (Genehunter NPL= 2.12; P = 0.014 and ASPEX mlod= 1.53). Within this region, the inventors focused their study on the *ASMT* gene using a combination of polymorphisms located in the promoters and intron 3 and adding 22 new families (Figure 1a, Table 1). Consistent with the initial fine mapping results, the inventors observed an increased of *ASMT* allelic sharing in affected sib pairs (Genehunter NPL= 2.95; P = 0.0014 and ASPEX mlod= 2.29; 0.0012). When families with only males affected sib pairs are used in the analysis, the linkage is dramatically increased (Genehunter NPL=4.8; P=4.86X10⁻⁷ and ASPEX mlod=5.92; P=1.81X10⁻⁷). However, in linkage studies of PAR1, the excess of paternal sharing should be taken with great care since this region is not fully independent from the sex chromosome segregation. This distortion effect specific to the paternal meiosis could account for the excess of sharing in male-male ASP and of non-sharing in male-female ASP. Nevertheless, the significant excess of maternal allelic sharing of *ASMT* in affected sib pairs (ASPEX maternal mlod= 1.9; P= 0.003) prompt the inventors to analyse the genetic variability of the *ASMT* gene in ASD compared to the control population.

### Genetic variability of the ASMT gene in ASD, ADHD and controls

To determine whether frequent variations in *ASMT* significantly associate with ASD, the inventors genotyped one insertion/deletion located in the promoter A and twelve Single Nucleotide Polymorphisms (SNPs) in 275 ASD, 95 ADHD and 187 geographically-matched controls. The genotype region covers 41.3 kb, including the coding exons and the two promoters (figure 1b). The *ASMT* gene can be divided in five haplotype blocks of high inter-marker linkage disequilibrium (D'>0.8). Comparison of allelic, and genotype frequencies are presented in figure 1c and table 2. Overall, a significant association was observed between ASD and one SNP rs4446909 located in the promoter B (P= 0.028). When the French and the Scandinavian samples are studied separately, a trend for association with rs4446909 is observed in the French sample (P=0.078) and a significant association with a close marker rs5989681 is present in the Scandinavian sample (P=0.047). The haplotype analysis indicated that one haplotype ACGC (including three SNPs in the promoter and one in the 5'UTR) was significantly less frequent in ASD (P=0.012) (Table 3). When populations are studied separately, although not significant, this haplotype was less frequent in both the French (P=0.057) and the Scandinavian ASD population (P=0.067) compared to their related control groups. In contrast, the GGGC haplotype could represent an "at risk haplotype" since it was more frequently observed in ASD (P=0.097). The same SNPs were genotyped in 95 Swedish individuals with ADHD, but no significant association was found. However, when individuals with ASD or ADHD were pooled and compared with controls, the association was more significant (rs4446909 P=0.015, ACGC, P=0.0077; GGGC, P= 0.042).

### Correlation between ASMT genotype and transcript level

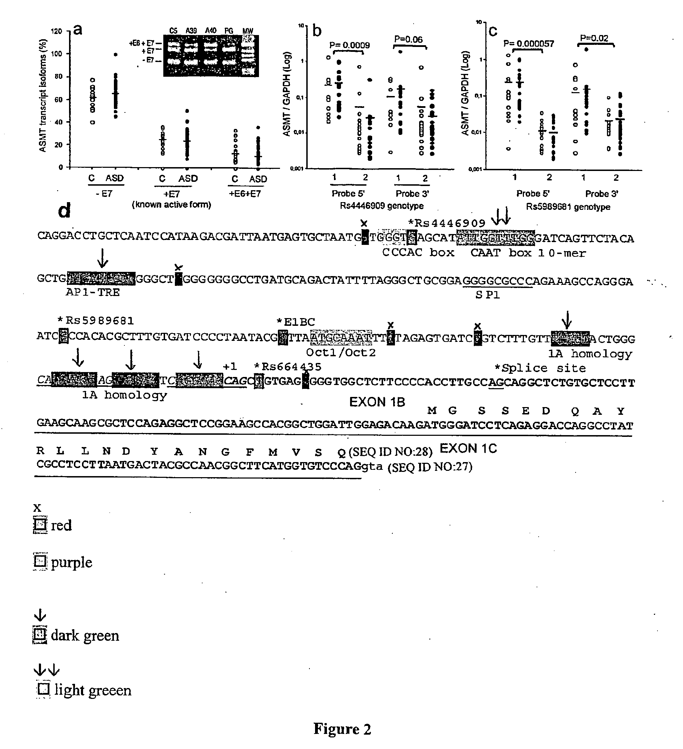

The *ASMT* gene contains two promoters (A and B) and two alternative spliced exons (Exon 6 and 7) (figure 2a). Promoter A was previously shown to be exclusive to retina ²⁷. Consistent with this, the inventors could not amplify *ASMT* transcripts originated from promoter A using human B lymphoblastoid cell lines (BLCL) or pineal gland cDNA. In contrast, the inventors could detect the three known alternative isoforms derived from promoter B (figure 2b insert). The long isoform contains all exons including exon 6, which is a Long Interspersed Nuclear Element (LINE). The medium isoform does not contain the LINE insertion and codes for the isoform homologous to the ASMT protein of other species. The shortest isoform does not contain the LINE insertion and exon 7. Only the medium isoform is supposed to be functional since the insertion of exon 6 or the deletion of exon 7 greatly modify the O-methylase domain of ASMT. Using fluorescent RT-PCR, the inventors could ascertain the relative proportion of the three *ASMT* isoforms. Overall, there was no significant difference in the abundance of the three isoforms between ASD and controls (figure 2b). However, in several individuals (13/48 patients and 4/23 controls), the relative percentage of the medium isoform was relatively low (<20%). For example, in the individual ASD49, the long *ASMT* isoform including the LINE was repetitively more abundant than the medium isoform (Figure 2b insert). Interestingly, one non synonymous SNP rs6588809 (R190W), located in exon6 within the LINE element, was associated with the level of LINE insertion (P=0.004). In average, individuals homozygotes for the C allele had 3.6 fold more insertion of the LINE in the *ASMT* transcript compared to individuals homozygotes for the T allele. This variation rs6588809 is probably located in one exonic splicing enhancer (ESE), a sequence which binds to splicing factors such as SR proteins. Despite, its functional consequence on the relative abundance of ASMT isoforms, no association was observed between rs6588809 and ASD.

To quantify the level of *ASMT* transcripts, the TaqMan technology and two independent probes E1CE2 and E8E9 located in the 5' and the 3' end of the mRNA respectively were used. No significant difference in *ASMT* mRNA level was observed between our sample of 60 ASD and 35 control individuals (figure 2c). In both samples, the *ASMT* transcript level was heterogenous ranging from 0.0019 to 1.39 compared to *GAPDH* mRNA level. However, when the *ASMT* genotypes and transcript levels were compared, the inventors could observe a very significant association between the transcript level and two SNPs rs4446909 (5'Probe P= 0.0009; 3' probe P=0.06) and rs5989681 (5'probe P= 0.000057; 3'probe P=0.02). These SNPs are distant from 109 bp, in high linkage disequilibrium (D'= 0.94) and located in promoter B (figure 2d). The SNP rs4446909 is situated at -207 bp from the transcription site in a CCCAC box and six nucleotides downstream a CAAT/10 mer box also present in the promoter A (Figure 2d). SNP rs5989681 is located at -97 bp from the transcription site in a putative binding site for the transcription factor NF-kappaB. Individuals homozygote for the G allele of both SNPs had less *ASMT* transcripts compared to heterozygotes individuals. Two individuals with a rs4446909 G/G genotype and a rs5989681 C/G genotype had a high transcript level, suggesting that the C allele of rs5989681 could be responsible for the high transcript level. Interestingly, these two SNPs (rs4446909 and rs5989681) were the one associated with ASD. The G alleles of both SNPs were more frequent in ASD and ADHD and were associated to a low *ASMT* transcript level. To explore further the relation between the *ASMT* gene and the susceptibility to these disorders, the inventors measured the enzyme activity in families with ASD and controls.

### ASMT activity in ASD and control individuals.

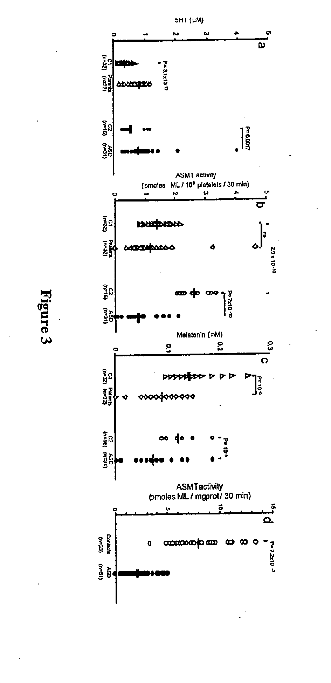

The ASMT activity was first investigated in the blood platelets of ASD, their parents and control individuals. As already reported in other samples, the inventors could observe an increase of serotonin concentration in the blood of ASD (5HT 1.0 ± 0.65 µM; P=1.46x10⁻⁷) and their parents (5HT 0.80 ± 0.24 µM ; P=1.85x10⁻¹⁰) compared to controls (5HT: 0.42 ± 0.22 µM; Figure 3a). Remarkably, the inventors also observed a strong decrease of ASMT activity for almost all of the individuals with ASD (ASMT blood 0.77 ± 0.47 pmoles/10⁹ platelets/30 min P=1.15x10⁻¹⁰) and their parents +(1.18± 0.87 pmoles/10⁹ platelets/30 min P=0.00045) compared to control (ASMT blood 1.81 ± 0.68 pmoles/10⁹ platelets/30 min ; Figure 3b). In parents, the ASMT activity was significantly decreased in the mothers (P=0.016), but not in the fathers (P=0.80) compared to sex- and age-matched controls. The decrease in ASMT was accompanied by a significant decrease of blood melatonin concentration in ASD (ML 0.07 ± 0.04 nM; P= 5.2x10⁻¹⁰) and their parents (ML 0.09 ± 0.04 nM; P= 2.04x10⁻⁶) compared to controls (ML 0.14 ± 0.04 nM) (Figure 3C). In parents, the melatonin concentration was significantly lower in mother (P=3.35x10-5) and in the fathers (P=0.007). To replicate these results, BLCLs from 51 individuals with ASD (11 already analysed in the blood sample) and 33 new independent controls were analysed (Figure 3d). Consistent, with the results obtained using the blood platelets, the level of ASMT activity was dramatically decreased in ASD (ASMT BLCL 3.29 ± 2.5 pmoles/mg prot/30 min) compared to controls (ASMT BLCL 7.97 ± 2.5 pmoles/mg prot/30 min; P= 7.2x10⁻⁷).

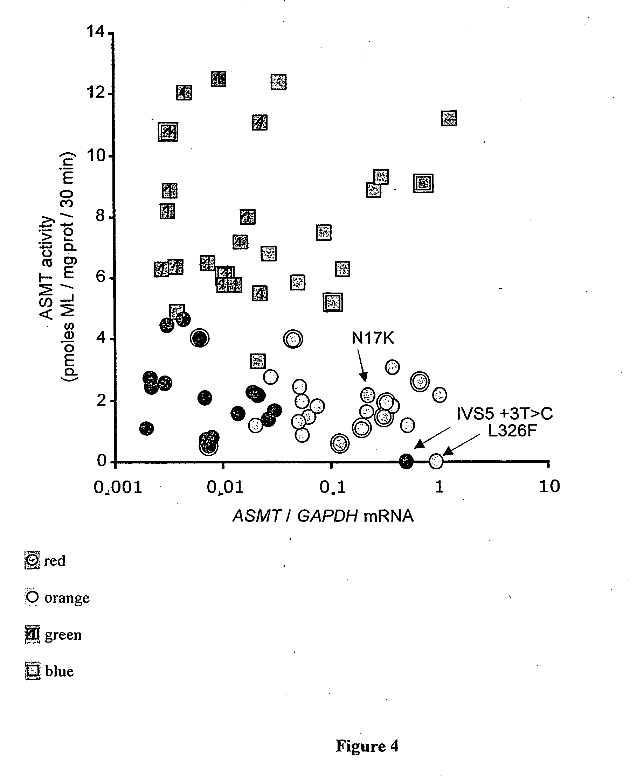

The inventors next plotted the relative quantity of *ASMT* mRNA against the level of the enzyme activity (Figure 4). In the control sample, the inventors could not detect a significant correlation between the RNA amount and the enzyme activity, suggesting that the level of *ASMT* transcript is not a strong limiting factor for enzyme activity in BLCL. In the ASD sample, the ASMT activity was decreased in almost all of the individuals, independently of their transcript level. For several individuals with ASD, the inventors cannot exclude that the low ASMT activity could be the direct consequence of a very low *ASMT* transcript level and/or a specific strong decrease in the medium isoform. Nevertheless, these results indicated that the global decrease of ASMT activity observed in ASD could not be explained solely by a decrease in *ASMT* mRNA. To further investigate this enzymatic deficiency, we screen the *ASMT* gene for mutation.

### Mutation screening of the ASMT gene in ASD, ADHD and controls

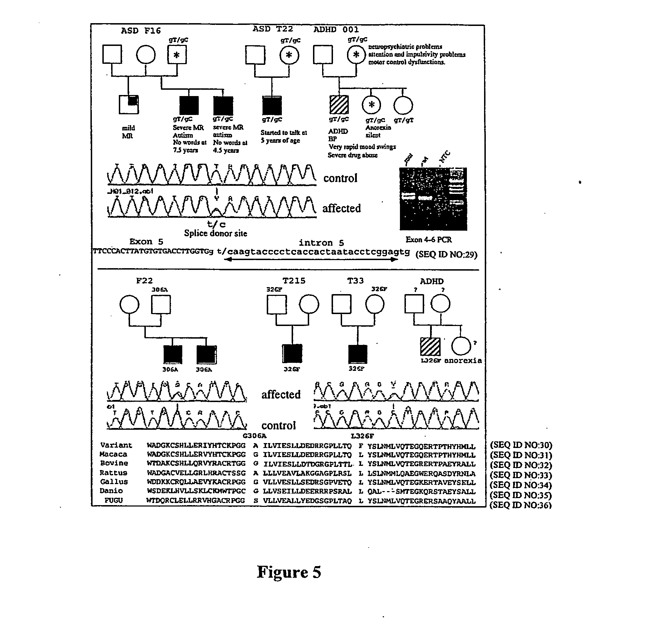

All exons of the ASMT gene, including the two promoters and alternatively spliced exons were directly sequenced in individuals with ASD (n = 275) or ADHD (n = 103). Several genetic variants were identified including a splice site mutation (IVS5+2T>C), five rare non-synonymous variations (N17K, K81E, R210H, G306A, L326F) and two synonymous variation (N 167N, Q205Q). The location, the segregation and the frequency of these rare variations are indicated in figure 5 and table 4. The splice site mutation (IVS5+2T>C) was present in two families with ASD and one with ADHD, but never observed in 411 controls. In one patient carrying the splice site mutation (IVS5+2T>C), the inventors could detect additional transcripts isoforms compared to controls, which originate from a donor splice site located 31 bp in intron 5 (figure 5). This abnormal spliced transcripts leads to different C-terminal protein sequences after amino acid G188 and premature truncation of all *ASMT* isoforms (figure 5). In ASD family 1, the mother transmitted the mutation to her son, who was homozygote for the G alleles of rs4446909 and rs5989681. In ASD family 2, the father transmitted the splice site mutation to his two sons with autism. The mother had previously a first child with autism but with a milder phenotype and from a different father. The haplotype analysis showed that the mother has transmitted the same *ASMT* GGGT promoter haplotype to all her affected sons. In the ADHD family 1, the mother carrying the splice site mutation had history of neuropsychiatric disorders including attention and impulsivity problems. She transmitted the mutation to her son with ADHD and to her daughter with anorexia, but not to her unaffected daughter. All individuals carried the haplotype. The N17K variation was observed in two ASD families (family 3 and 4) with parents originated from Asiatic countries. In family 3 and 4, the mother transmitted the mutation. In ASD family 5, the mother transmitted the non-synonymous variation K81E. In ASD family 6 from Norway, the father transmitted a variation G306A to his two sons with autism. The variation L326F was identified in two independent families with ASD and one with ADHD. In family 7 and 8, the mutations were transmitted by the mother and the father, respectively. In ADHD family 2, the son with ADHD has a 326 mutation and there is a daughter with anorexia. None of these non-synonymous variations were observed in controls except L326F, which was present in 3/400 controls. To ascertain the functional effects of these rare variants, the inventors measured the *ASMT* enzyme activity and the melatonin concentration in the carriers.

### Biochemical and clinical characterisation of the individuals carrying rare variations of the ASMT gene.

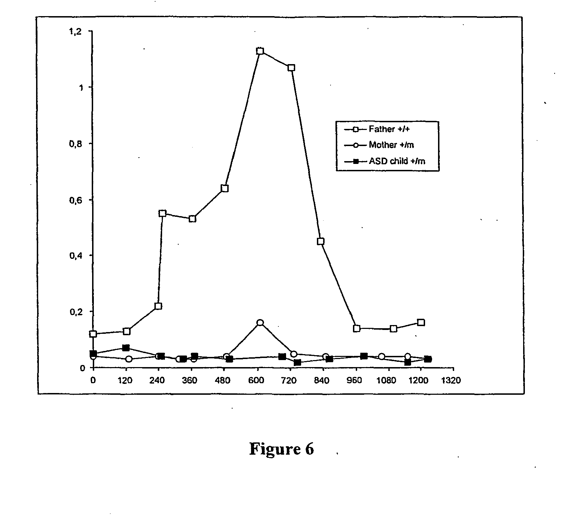

Biochemical analyses of the blood platelets indicate that individuals carrying the splice site mutation (IVS5+2T>C) or the L326F mutation have low HIOMT activity, a decrease of melatonin and a relatively high concentration of 5-HT compared to controls (Table 5). Consistent with the results obtained on the blood samples, ASMT activity in BLCL was found reduced in all individuals carrying the (IVS5+2T>C) and the L326F mutations. In these individuals, the melatonin concentration was also found decreased compared to controls. To further explore the ASMT deficit *in vivo,* the inventors investigated family 1 carrying the splice site mutation (figure 6). The father does not carry the mutation and showed a normal increase of melatonin during the night. In contrast, both carriers of the *ASMT* splice mutation, the unaffected mother and the son with ASD, showed no increase of melatonin during the night. Despite this absence of melatonin, no obvious difference in the sleep pattern was observed compared to controls.

### Mutations of AANAT in individuals with ASD and ADHD

In the melatonin biosynthesis pathway, the enzyme AANAT is located before the ASMT and transform the serotonin into N-acetyl serotonin. Mutations in the AANAT gene were screened and five rare non-synonymous variations (T3M, A13S, V621, A157V, A163V) were indetified in individuals with ASD and ADHD. These variations were not observed in 200 controls.

### EXAMPLE 2

### Mutations in the melatonin receptors MTNR1A and MTNR1B decrease melatonin binding and confer susceptibility to psychiatric disorders.

During the search of susceptibility factors to autism or Attention Deficits and Hyper Activity Disorder (ADHD), the inventors identified genetic mutations in the ASMT (Acetyl serotonin methyl transferase) gene (see Example 1). In this present example, the inventors report genetic variations in the melatonin receptors including a stop mutation in a patient with ADHD, which is absent from all controls tested (n=150). In addition, the inventors identified an ASMT splice site mutation in a patient with OCD. These results confirm the results of Example 1 showing that the melatonin pathway is associated to psychioatric disorder.

### Results

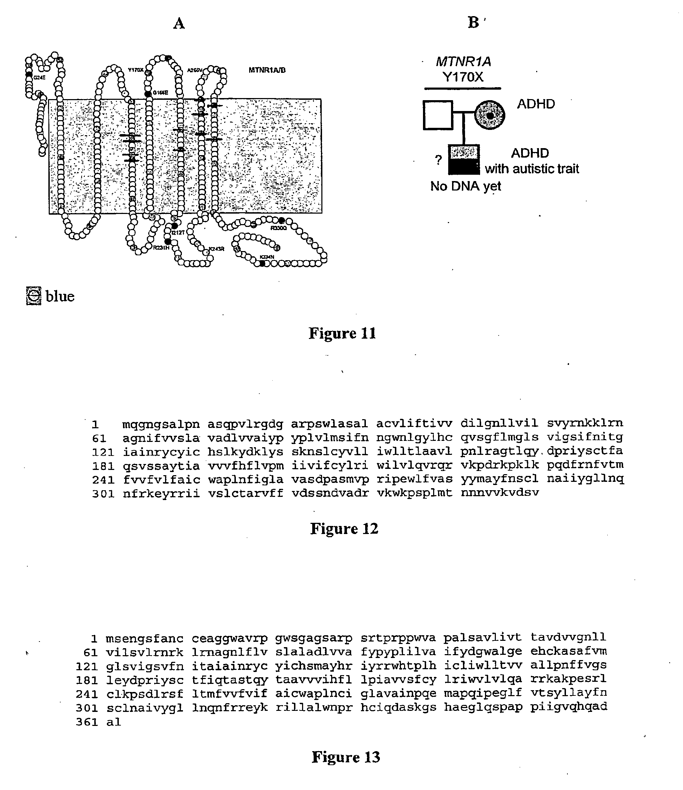

The inventors investigated whether variations in *MTNR1A* and *MTNR1B* were associated with ASD and ADHD, by directly sequencing all exons and the promoters of individuals with ASD (n=78-130) or ADHD (n=79-102). Several exonic mutations were identified (Table 9, Figure 11A), including a stop mutation in a patient with ADHD, caused by a point mutation at position 170 (see SEQ ID NO: 5), namely a Y170X mutation. This woman has a son suffering from ADHD with autistic traits, but no DNA is available for this patient.

On the basis of the results shown in Example 1, the inventors have now evidenced that the melatonin receptor MTNR1A can also be mutated in individuals with ADHD. These results confirm that abnormal melatonin pathway is a risk factor for neuropsychiatric disorders.

### General conclusions

Melatonin is synthesised in two steps from the neurotransmitter serotonin. The HIOMT enzyme catalyses the final reaction transforming N-acetylserotonin into melatonin. This hormone is mainly produced in the pineal gland but also in other tissues such as retina or lymphocytes. It is secreted during the circadian cycle with high level during the night and low level during the day. The physiological role of melatonin is still unclear but it plays crucial roles correlated to the circadian or seasonal rhythms. Furthermore, melatonin was shown to have anti-oxidative or anti-aging properties as well as effects on hormonal autocrine/paracrine functions, on the immune system, on the modulation of neurotransmitter release.

Sleep problems are frequently reported in patients with neuro-psychiatric disorders. In addition, an increase concentration of serotonin in individuals with autism as well as in there relatives is one of the most replicated finding in autism. However, despite numerous genetic studies on the serotonin transporters or receptors, the reason why there is high level of serotonin was never elucidated. The outcomes of the present invention are extremely important for the diagnostic and therapy of autism and ADHD for at least three major reasons:
A. The biochemical analysis of the melatonin biosynthesis is the first biological marker associated with susceptibility to neuropsychiatric disorders (autism, ADHD and OCD). This assay could be used as a screening test since concentration of melatonin could be quantified with a simple saliva sample and the HIOMT enzymatic activity can be measured using a blood sample.
B. In the case of a decrease melatonin concentration is identified in one patient, the supplementation with exogenous melatonin is feasible and could correct the genetic deficit.
C. The identification of these mutations should permit to find others susceptibility factors having roles in the same biological pathway.

**Table 1 ASMT allelic sharing in ASD sib-pairs families**

| | IBD status | | | | | | Parental sharing % (IBD1:1BD0)* | | | |
|---|---|---|---|---|---|---|---|---|---|---|
| | 0 | M1 | P1 | 2 | P value | | Maternal | P value | Paternal | P value |
| Affected | | | | | | | | | | |
| sib pairs | | | | | | | | | | |
| Male-Male | 3 | 4 | 5 | 15 | **0.0033** | | 71 (20:8) | **0.023** | 75 (24:8) | **0.0047** |
| Male- | 8 | 8 | 1 | 5 | 0.11 | | 60 (15:10) | 0.31 | 31 (6:18) | 0.014 |
| Female | | | | | | | | | | |
| Female- | 1 | 0 | 1 | 1 | - | | 33 (1:2) | - | 75 (3:1) | - |
| Female | | | | | | | | | | |
| All | 12 | 12 | 7 | 21 | **0.049** | | 64 (36:20) | **0.033** | 62 (33:27) | 0.44 |

| | | | | | | | | | | |
|---|---|---|---|---|---|---|---|---|---|---|
| * Thirteen additional families informative for only one of the parent were included in the parental sharing compare to the IBD status. | | | | | | | | | | |

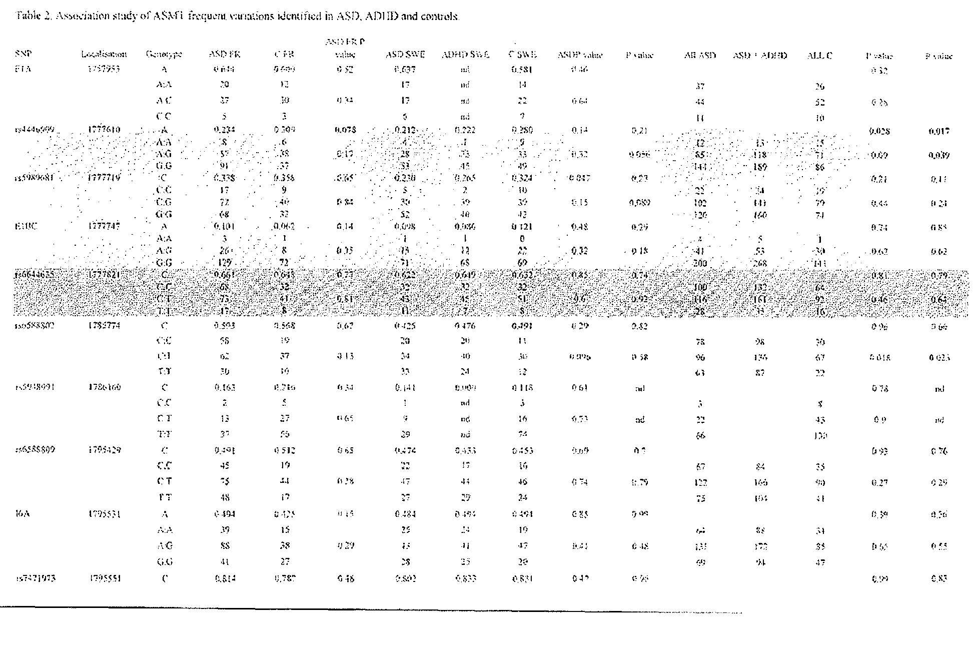

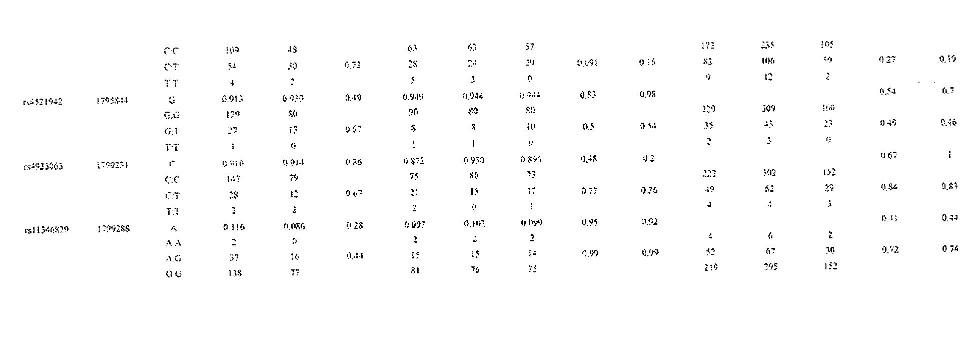

**Table 3. Haphotypic association using the four frequent polymorphisms located in the promoter B and 5' UT5 of ASMT**

| | French sample (%) | | | | Scandinavian sample (%) | | | | | | Combined sample | |
|---|---|---|---|---|---|---|---|---|---|---|---|---|
| | ASD | Control | P values | | ASD | ADHD | Controls | ASD P value | ADHD P value | | ASD P value | ASD-ADHD P value |
| GGGC | 0.308 | 0.279 | 0 .51 | | 0.396 | 0.374 | 0.313 | 0.099 | 0.058 | | 0.096 | 0.042 |
| ACGC | 0.225 | 0.304 | 0.057 | | 0.185 | 0.214 | 0.267 | 0.067 | 0.24 | | 0.012 | 0.0077 |
| GGGC | 0.242 | 0.306 | 0.13 | | 0.272 | 0.267 | 0.241 | 0.54 | 0.90 | | 0.43 | 0.47 |
| GCGC | 0.112 | 0.052 | 0.022 | | 0.017 | 0.051 | 0.037 | 0.26 | 0.82 | | 0.086 | 0.17 |
| GGAT | 0.092 | 0.054 | 0.14 | | 0.098 | 0.078 | 0.108 | 075 | 0.25 | | 0.68 | 0.76 |
| GCGT | 0 | 0 | - | | 0.013 | 0 | 0.013 | 0.99 | - | | - | - |
| AGGC | 0 | 0 | - | | 0.018 | 0 | 0.011 | 0.59 | - | | 0.32 | - |

**Table 4. ASMT Rare variations identified in ASD, ADHD and controls.**

| **Exon/Intron** | **Variant** | **Nucleotide*** | **French ASD** | **Swedish ASD** | **Swedish ADHD** | **French Control** | **Swedish Control** | **th Africa Cont** | **All controls** |
|---|---|---|---|---|---|---|---|---|---|
| 5'UTR | | C/T | 0\|136 | 0\|82 | 0\|103 | 0\|84 | 1\|90 | Nd | 1/174 |
| E1A | | A/g | 2\|136 | 0\|82 | 0\|103 | 0\|84 | 0\|90 | Nd | 0/174 |
| E1A | | G/C | 2\|136 | 0\|82 | 0\|103 | 0\|84 | 0\|90 | Nd | 0/174 |
| E1A | | A/T | 2\|136 | 0\|82 | 0\|103 | 0\|84 | 0\|90 | Nd | 0/174 |
| E1A | | A/g | 2\|136 | 0\|82 | 0\|103 | 0\|84 | 0\|90 | Nd | 0/174 |
| E1A | | C/T | 1\|136 | 0\|82 | 0\|103 | 0\|84 | 0\|90 | Nd | 0/174 |
| E1A | | C/A | 0\|136 | 1\|82 | 0\|103 | 0\|84 | 0\|90 | Nd | 0/174 |
| E1B | N17K | C232A | 1\|140 | 1\|80 | 0\|103 | 0\|79 | 0\|92 | Nd | 0/171 |
| E1B | | A/T | 1\|140 | 0\|80 | 0\|103 | 0\|79 | 0\|92 | Nd | 0/171 |
| E1B | | C/T | 2\|140 | 0\|80 | 0\|103 | 0\|79 | 0\|92 | Nd | 0/171 |
| E1B | | C/T | 1\|140 | 1\|80 | 0\|103 | 0\|79 | 0\|92 | Nd | 0/171 |
| E1B | | C/A | 0\|140 | 1\|80 | 0\|103 | 0\|79 | 0\|92 | Nd | 0/171 |
| E2 | K81E | A422G | 1\|150 | 0\|86 | 0\|103 | 0\|67 | 0\|53 | Nd | 0/120 |
| INTRON 2 | | C/A | 1\|150 | 0\|86 | 0\|103 | 0\|67 | 0\|53 | Nd | 0/120 |
| INTRON 2 | | G/A | 3\|150 | 0\|86 | 0\|103 | 0\|67 | 0\|53 | Nd | 0/120 |
| INTRON 4 | | C/T | 1\|174 | 0\|97 | 0\|103 | 0\|94 | 0\|93 | Nd | 0/187 |
| E5 | N167N | | 0\|182 | 1\|102 | 0\|103 | 0\|94 | 0\|145 | 0\|172 | 0/411 |
| INTRON 5 | IV85+2T>C | T/C | 2\|182 | 0\|102 | 1\|102 | 0\|94 | 0\|145 | 0\|172 | 0/411 |
| INTRON 6 | | G/A | 1\|168 | 0\|95 | 0\|103 | 0\|80 | 0\|87 | Nd | 0/167 |
| E7 | D238G | A894G | 0\|166 | 0\|97 | 0\|103 | 0\|94 | 0\|97 | Nd | 1/191 |
| E7 | K247R | A921G | 0\|116 | 0\|97 | 0\|103 | 0\|94 | 0\|97 | Nd | 1/191 |
| INTRON 7 | | C/T | 0\|166 | 0\|97 | 0\|103 | 0\|94 | 1\|97 | Nd | 1/191 |
| E8 | P271L | G994T | 0\|176 | 0\|96 | 0\|103 | 0\|94 | 1\|93 | Nd | 1/187 |
| E8 | C301S | G1083C | 0\|176 | 0\|96 | 0\|103 | 0\|94 | 0\|93 | Nd | 1/187 |
| E9 | G306A | G1098C | 0\|186 | 1\|101 | 0\|103 | 0\|88 | 0\|145 | 0\|95 | 0/328 |
| | R319Q | G1137A | 0\|186 | 0\|101 | 0\|103 | 0\|88 | 0\|145 | 0\|95 | 0/328 |
| | L326F | C1157T | 1\|186 | 1\|101 | 1\|103 | 0\|88 | 2\|145 | 1\|95 | 3/328 |

| | | | | | | | | | |
|---|---|---|---|---|---|---|---|---|---|
| * reference sequence Genebank NM004043 | | | | | | | | | |

**Table 5. Biochemical measurement in blood and BLCL from families carrying rare variations**

| | Axe I Diagnosis | | ASMT genotype | | Blood sample | | | | BLCL | |
|---|---|---|---|---|---|---|---|---|---|---|
| | | | | | 5-HT (µM) | HIOMT (pmoles/10⁹ platelets/30 min) | ML (nM) | | HIOMT (pmoles/ mg prot/30 min) | ML (pM) |
| Father | Nd | | +/+ | | **1.14** | 0.96 | 0.12 | | - | |
| Mother | Nd | | +/(IVS5+2T >C) | | **1.18** | **0.39** | **0.05** | | - | |
| Proband I | Autism | | +/(IVS5+2T >C) | | 0.89 | **0.22** | **0.04** | | **<0.01** | **<0.02** |
| Father | Nd | | +/+ | | 0.90 | 0.88 | **0.09** | | - | - |
| Mother | Nd | | +/L326F | | 0.96 | **<0.06** | **<0.02** | | - | - |
| Proband 2 | Autism | | -/L326F | | 0.80 | **<0.06** | **<0.02** | | **<0.01** | **<0.02** |
| Controls range (n=47) | | | | | 0.12-1.17 | 0.86-3.36 | 0.1-0.26 | | 3.3-13.4 | 2.3-10 |

**Table 6. Genetic variations of the AANAT gene identified in individuals with ASD, ADHD and controls.**

| | Localisation* | ASD (n=287) | ADHD (n=96) | Controls (n=165) |
|---|---|---|---|---|
| Promoter | TG | 169 :179 | | 170 :164 |
| | G | 179 :169 | | 180 :140 |
| 5'UTR | | | | |
| Intron1 | | | | |
| Exon2 | C242T | 3 | | 0 |
| | (T3M) | | | |
| | G271T | 1 | | 0 |
| | (A138) | | | |
| Intro 2 | GA | | | |
| Exon3 | G418A | 1 | 1 | 0 |
| | (V621) | | | |
| Intron 3 | | | | |
| Exon 4 | TC (H122H) | 1 | 0 | 0 |
| | CT (R128R) | 1 | 0 | 0 |
| | C689T | 0 | 1 | 0 |
| | (A152V) | | | |
| | CT (A156A) | 0 | 1 | 0 |
| | C704T | 1 | 0 | 0 |
| | (A157V) | | | |
| | C722T | 1 | 1 | 0 |
| | (A163V) | | | |
| | GA (G177D) | 0 | 0 | 1 |
| | CA (H8H) | 1 | 0 | 0 |
| 3'UTR | CT | | | |
| | GA | | | |

| | | | | |
|---|---|---|---|---|
| *reference sequence Genebank NM_001088 | | | | |

**Table 7. Allelic and haplotypic association using two AANAT frequent promoter polymorphisms**

| | French and Swedish ASD N=192 | P value | Swedish ADHD n=92 Major Allele Frequence P value | P value | French Controls n=95 | Swedish controls N=95 |
|---|---|---|---|---|---|---|
| Single | | | | | | |
| rs | G 0.51 | 0.54 | T 0.57 | 0.45 | G 0.512 | T 0.53 |
| rs | G 0.51 | 0.21 | G 0.57 | 0.45 | G 0.596 | G 0.53 |
| Haplotype | | | | | | |
| Rs-rs | | | | | | |
| TG | 0.433 | 0.17 | 0.52 | 0.68 | 0.481 | 0.499 |
| GC | 0.426 | 0.89 | 0.38 | 0.27 | 0.411 | 0.438 |
| GG | 0.086 | 0.42 | 0.05 | 0.405 | 0.101 | 0.032 |
| TC | 0.055 | 0.038 | 0.05 | 0.405 | 0 | 0.032 |

**Table 8. PCR and sequencing primers for Human ASMT**

| **EXON** | **Size (exon)** | **Primers** | **Size PCR (hp)** | **Ta (°C)** | **comment** | **Seq primer** |
|---|---|---|---|---|---|---|
| 1A | 78 | ASMT1AF (SEQ ID NO: 7) : GAGCGATTCTTCTGCCTCAGC | 875 | 67 | 5% DMSO | Fwd and rev |
| | | | | | | |
| | | ASMT1AR (SEQ ID NO: 8) : TCTGCGCACACTCCCAGGTG | | | (1m elong) | |
| 1B/C | 136 | ASMT1 BF (SEQ ID NO: 9) : GAGGCAGGAGAATCGCTTGAA | 820 | 67 | 5% DMSO | Fwd and rev |
| | | | | | | |
| | coding:69 | ASMT1 BR (SEQ ID NO: 10) : GGCTACATCGTGGGTGTACGTC | | | (1m elong) | |
| 2 | 175 | ASMT2F (SEQ ID NO: 11): TGGTGCAATCTCATTTGACTCTG | 552 | 58 | | Rev |
| | | | | | | |
| | | ASMT2R (SEQ ID NO: 12): GGGTTCATGCCATTCTCCTG | | | | |
| 3 | 130 | ASMT3F1 (SEQ ID NO: 13): CAGCTGTACAAGGCAAGAGGA | 950 | 64 | 5% DMSO | Fwd and rev |
| | | | | | | |
| | | ASMT3R2 (SEQ ID NO: 14) : CTTTCACCTCCTCCACTGCCA | | | (1m elong) | |
| 4 | 69 | ASMT4F (SEQ ID NO: 15) : GCCTGGGCTACAGAGCTGAAA | 283 | 55 | | Rev |
| | | | | | | |
| | | ASMT4R (SEQ ID NO: 16): CTCCTGGGTTGTGCCATTTG | | | | |
| 5 | 119 | ASMT5F (SEQ ID NO: 17) : CCTGTGGGGTATAGCTCCGTTC | 331 | 64 | | Fwd |
| | | | | | | |
| | | ASMT5R (SEQ ID NO: 18) : CGCACATGTCAAAGCATCAGA | | | | |
| 6 | 84 | ASMT6F (SEQ ID NO: 19) : AGCTTGCAGTGAGCGGAAATC | 342 | 64 | | Fwd |
| | | | | | | |
| | | ASMT6R (SEQ ID NO: 20) : GCACCCATCGACTCGTCATTT | | | | |
| 7 | 141 | ASMT7F (SEQ ID NO: 21) : TGGGTTGGACCCTTCATGAGT | 352 | 64 | | Rev |
| | | | | | | |
| | | ASMT7R (SEQ ID NO: 22) : GTGTTTCCGGGAGTGAGAGGA | | | | |
| 8 | 123 | ASMT8F (SEQ ID NO: 23) : AGCCTGGAAGACCTGGGAAAG | 338 | 64 | | Fwd |
| | | | | | | |
| | | ASMT8R (SEQ ID NO: 24) : CCTGTGGGATGATTTCAGTGC | | | | |
| 9 | 279 | ASMT9F (SEQ ID NO: 25) : GGTGCCCTGACTGTCCTCTGA | 506 | 64 | | Fwd |
| | | | | | | |
| | coding:212 | ASMT9R (SEQ ID NO: 26) : CCATCAGCGTGGTCCTCAGTA | | | | |
| All PCRs performed with Qiagen HotstarTaq with the temperature profile: 15 min at 95° 35 cycles of: 30s at 95° 30s at Ta. 30s-1min at 72° followed by a final extension step of 10 min at 72°. | | | | | | |

**Table 9. Variations identified in ASD, ADHD and controls**

| | ***MTNR1A*** | | | | ***MTNR1B*** | | | |
|---|---|---|---|---|---|---|---|---|
| ***Rares variants*** | ***ASD*** | ***ADHD*** | ***Controls*** | | ***Rares variants*** | ***ASD*** | ***ADHD*** | ***Controls*** |
| Gly166 Glu | 4/79 | 1/87 | 5/100 | | Gly24Glu | 8/78 | 8/62 | N/A |
| | | | | | | 2/78 HO | 1/62 HO | |
| **Tyr170X** | **0/79** | **1/79** | **0/100** | | Arg 231 His | 2/130 | 2/99 | N/A |
| Ile189IIe* | 2/79 | 8/87 | 0/100 5/100 | | Lys 243 Arg | 8/130 | 3/102 | N/A |
| | | 2/87 HO | | | | | | |
| Ile 212Thr | 2/79 | 0/87 | 0/100 | | Arg330 Gln | 1/130 | 0/102 | N/A |
| Ala266Val | 2/79 | 4/87 | 4/100 | | | | | |
| Arg308Arg | 3/79 | 5/87 | 2/100 | | | | | |
| | 1/79 HO | | | | | | | |
| Thr315Thr | 5/79 | 5/87 | 2/100 | | | | | |
| | 1/79 HO | | | | | | | |
| Lys334Asn | 1/79 | 2/87 | 2/100 | | | | | |

| | | | | | | | | |
|---|---|---|---|---|---|---|---|---|
| *P= 0.05 between ADHD and Controls ; HO : homozygotes; N/A: not available | | | | | | | | |

### References

1. Folstein, S. E. & Rosen-Sheidley, B. Genetics of autism: complex aetiology for a heterogeneous disorder. Nat Rev Genet 2, 943-955 (2001).
2. Veenstra-VanderWeele, J. & Cook, E. H. Molecular genetics of autism spectrum disorder. Mol Psychiatry 9, 819-832 (2004).
3. Bobb, A. J., Castellanos, F. X., Addington, A. M. & Rapoport, J. L. Molecular genetic studies of ADHD: 1991 to 2004. Am J Med Genet B Neuropsychiatr Genet 132, 109-25 (2005).
4. Faraone, S. V. et al. Molecular genetics of attention-deficit/hyperactivity disorder. Bioi Psychiatry 57, 1313-23 (2005).
5. American Psychiatric Association. Diagnostic and Statistical Manual of Mental Disorders, 4th Ed. (American Psychiatric Press, Washington D.C., 1994).
6. Biederman, J. & Faraone, S. V. Attention-deficit hyperactivity disorder. Lancet 366, 237-48 (2005).
7. Smalley, S. L. Genetic influences in childhood-onset psychiatric disorders: autism and attention-deficit/hyperactivity disorder. American Journal of Human Genetics 60, 1276-82. (1997).
8. Ozonoff, S. & Jensen, J. Brief report: specific executive function profiles in three neurodevelopmental disorders. J Autism Dev Disord 29, 171-7 (1999).
9. Handen, B. L., Johnson, C. R. & Lubetsky, M. Efficacy of methylphenidate among children with autism and symptoms of attention-deficit hyperactivity disorder. J Autism Dev Disord 30, 245-55 (2000).
10. Smalley, S. L. et al. Genetic linkage of attention-deficit/hyperactivity disorder on chromosome 16p13, in a region implicated in autism. Am J Hum Genet 71, 959-63 (2002).
11. Thomas, N. S. et al. Xp deletions associated with autism in three females. Human Genetics 104, 43-48 (1999).
12. Boycott, K. M. et al. A familial contiguous gene deletion syndrome at Xp22.3 characterized by severe learning disabilities and ADHD. Am J Med Genet A 122, 139-47 (2003).
13. Doherty, M. J. et al. An Xp; Yq translocation causing a novel contiguous gene syndrome in brothers with generalized epilepsy, ichthyosis, and attention deficits. Epilepsia 44, 1529-35 (2003).
14. Yi, H., Donohue, S. J., Klein, D. C. & McBride, O. W. Localization of the hydroxyindole-O-methyltransferase gene to the pseudoautosomal region: implications for mapping of psychiatric disorders. Hum Mol Genet 2, 127-31 (1993).
15. Reiter, R. J. Melatonin: clinical relevance. Best Pract Res Clin Endocrinol Metab 17, 273-85 (2003).
16. Simonneaux, V. & Ribelayga, C. Generation of the melatonin endocrine message in mammals: a review of the complex regulation of melatonin synthesis by norepinephrine, peptides, and other pineal transmitters. Pharmacol Rev 55, 325-95 (2003).
17. Reiter, R. J. Pineal melatonin: cell biology of its synthesis and of its physiological interactions. Endocr Rev 12, 151-80 (1991).
18. Nelson, R. J. & Drazen, D. L. Melatonin mediates seasonal changes in immune function. Ann N Y Acad Sci 917, 404-15 (2000).
19. Nir, I. et al. Brief report: circadian melatonin, thyroid-stimulating hormone, prolactin, and cortisol levels in serum of young adults with autism. J Autism Dev Disord 25, 641-54 (1995).
20. Lord, C. What is melatonin? Is it a useful treatment for sleep problems in autism? J Autism Dev Disord 28, 345-6 (1998).
21. Hayashi, E. Effect of melatonin on sleep-wake rhythm: the sleep diary of an autistic male. Psychiatry Clin Neurosci 54, 383-4 (2000).
22. Kulman, G. et al. Evidence of pineal endocrine hypofunction in autistic children. Neuroendocrinol Lett 21, 31-34 (2000).
23. Pacchierotti, C., lapichino, S., Bossini, L., Pieraccini, F. & Castrogiovanni, P. Melatonin in psychiatric disorders: a review on the melatonin involvement in psychiatry. Front Neuroendocrinol 22, 18-32 (2001).
24. Yun, A. J., Bazar, K. A. & Lee, P. Y. Pineal attrition, loss of cognitive plasticity, and onset of puberty during the teen years: is it a modern maladaptation exposed by evolutionary displacement? Med Hypotheses 63, 939-50 (2004).
25. Tordjman, S., Anderson, G. M., Pichard, N., Charbuy, H. & Touitou, Y. Nocturnal excretion of 6-sulphatoxymelatonin in children and adolescents with autistic disorder. Biol Psychiatry 57, 134-8 (2005).
26. Ishizaki, A., Sugama, M, & Takeuchi, N. [Usefulness of melatonin for developmental sleep and emotional/behavior disorders--studies of melatonin trial on 50 patients with developmental disorders]. No To Hattatsu 31, 428-37 (1999).
27. Rodriguez, I. R., Mazuruk, K., Schoen, T. J. & Chader, G. J. Structural analysis of the human hydroxyindole-O-methyltransferase gene. Presence of two distinct promoters. J Biol Chem 269, 31969-77 (1994).
28. Chanut E, Nguyen-Legros J, Versaux-Botteri C, Trouvin JH, Launay JM. Determination of melatonin in rat pineal, plasma and retina by high-performance liquid chromatography with electrochemical detection. J Chromatogr B Biomed Sci Appl. 1998 May 8;709(1):11-8
29. Finocchiaro LM, Nahmod VE, Launay JM. Melatonin biosynthesis and metabolism in peripheral blood mononuclear leucocytes. Biochem J. 1991 Dec 15;280 (Pt 3):727-31.

## Claims

1. A method for determining the likelihood of an individual developing a psychiatric disorder selected from the group autism spectrum disorders (ASD), Attention Deficits and Hyper Activity Disorder (ADHD); comprising:
- identifying at least one genetic variation in a gene selected from the group consisting of ASMT, AANAT, involved in the modulation of melatonin and/or
- assaying the enzymatic activity of an enzyme encoded by said ASMT gene involved in the melatonin biosynthesis pathway in a sample of said individual,
said method may also comprise the step of:
- assaying the melatonin level,
whereby identification of said at least one genetic variation and/or decrease in said enzymatic activity as compared to a psychiatric-disorder free individual, is indicative of a likelihood that said individual develops a psychiatric disorder.

2. The method of claim 1, **characterized in that** said genetic variation is a point mutation in said ASMT gene.

3. The method of claim 2, **characterized in that** said point mutation gives rise to a mutation at position 17, 81, 210, 306 or 326 as defined by the position in SEQ ID NO: 1,

4. The method of claim 3, **characterized in that** said mutation in SEQ ID NO:1 is a N17K mutation.

5. The method of claim 3, **characterized in that** said mutation in SEQ ID NO: is a K81 E mutation.

6. The method of claim 3, **characterized in that** said mutation in SEQ ID NO:1 is a R210H mutation.

7. The method of claim 3, **characterized in that** said mutation in SEQ ID NO:1 is a G306A mutation.

8. The method of claim 3, **characterized in that** said mutation in SEQ ID NO: 1 is a L326F mutation.

9. The method of claim 1, **characterized in that** said genetic variation is a splice site mutation of said ASMT gene.

10. The method of claim 9, **characterized in that** the splice site mutation is IVS5+2T>C.

11. The method of claim 1, **characterized in that** said genetic variation is located in a single nucleotide polymorphism (SNP) of the ASMT gene.

12. The method of claim 11, **characterized in that** said SNP is Rs5989681 or Rs6588809.

13. The method of claim 2, **characterized in that** said point mutation gives rise to a mutation at position 17, 81 or 298 as defined by the position in SEQ ID NO: 2.

14. The method of claim 2, **characterized in that** said point mutation gives rise to a mutation at position 17, 81, 231 or 251 as defined by the position in SEQ ID NO: 3.

15. The method of claim 1, **characterized in that** said genetic variation is a point mutation of said AANAT gene.

16. The method of claim 15, **characterized in that** said point mutation gives rise to a mutation at position 3, 13, 62, 157 or 163 as defined by the position in SEQ ID NO: 4.

17. The method of claim 16, **characterized in that** said mutation in SEQ ID NO:4 is a T3M mutation.

18. The method of claim 16, **characterized in that** said mutation in SEQ ID NO:4 is a A13S mutation.

19. The method of claim 16, **characterized in that** said mutation in SEQ ID NO:4 is a V62l mutation.

20. The method of claim 16, **characterized in that** said mutation in SEQ ID NO:4 is a A157V mutation.

21. The method of claim 16, **characterized in that** said mutation in SEQ ID NO:4 is a A163V mutation.

## Patentansprüche

1. Verfahren zur Bestimmung der Wahrscheinlichkeit dafür, dass ein Individuum eine psychiatrische Störung entwickelt, die ausgewählt ist aus der Gruppe der Störungen, die dem autistischen Spektrum zugeordnet sind (ASD), der Aufmerksamkeitsdefizit- und Hyperaktivität-Störung (ADHD); umfassend:
- Identifizieren mindestens einer genetischen Variation in einem Gen, das ausgewählt ist aus der Gruppe bestehend aus ASMT, AANAT und das an der Modulierung von Melatonin beteiligt ist, und/oder
- Analysieren der enzymatischen Aktivität eines Enzyms, das von dem am Melatonin-Biosyntheseweg beteiligten ASMT-Gen kodiert wird, in einer Probe aus dem Individuum,
wobei das Verfahren außerdem den folgenden Schritt umfasst:
- Analysieren des Melatoninspiegels,
- wobei die Identifizierung der mindestens einen genetischen Variation und/oder die Abnahme der enzymatischen Aktivität im Vergleich zu einem nicht von einer psychiatrischen Störung betroffenen Individuum auf eine Wahrscheinlichkeit hinweist, dass das Individuum eine psychiatrische Störung entwickeln wird.

2. Verfahren nach Anspruch 1, **dadurch gekennzeichnet, dass** die genetische Variation eine Punktmutation in dem ASMT-Gen ist.

3. Verfahren nach Anspruch 2, **dadurch gekennzeichnet, dass** die Punktmutation eine Mutation an der Position 17, 81, 210, 306 oder 326, definiert durch die Position in SEQ ID NO: 1, entstehen lässt.

4. Verfahren nach Anspruch 3, **dadurch gekennzeichnet, dass** die Mutation in SEQ ID NO: 1 eine N17K-Mutation ist.

5. Verfahren nach Anspruch 3, **dadurch gekennzeichnet, dass** die Mutation in SEQ ID NO: 1 eine K81 E-Mutation ist.

6. Verfahren nach Anspruch 3, **dadurch gekennzeichnet, dass** die Mutation in SEQ ID NO: 1 eine R210H-Mutation ist.

7. Verfahren nach Anspruch 3, **dadurch gekennzeichnet, dass** die Mutation in SEQ ID NO: 1 eine G306A-Mutation ist.

8. Verfahren nach Anspruch 3, **dadurch gekennzeichnet, dass** die Mutation in SEQ ID NO: 1 eine L326F-Mutation ist.

9. Verfahren nach Anspruch 1, **dadurch gekennzeichnet, dass** die genetische Variation eine Spleißstellenmutation des ASMT-Gens ist.

10. Verfahren nach Anspruch 9, **dadurch gekennzeichnet, dass** die Spleißstellenmutation IVS5+2T>C ist.

11. Verfahren nach Anspruch 1, **dadurch gekennzeichnet, dass** die genetische Variation in einem einzigen Nucleotid-Polymorphismus (SNP) des ASMT-Gens lokalisiert ist.

12. Verfahren nach Anspruch 11, **dadurch gekennzeichnet, dass** der SNP Rs5989681 oder Rs6588809 ist.

13. Verfahren nach Anspruch 2, **dadurch gekennzeichnet, dass** die Punktmutation eine Mutation an der Position 17, 81 oder 298, definiert durch die Position in SEQ ID NO: 2, entstehen lässt.

14. Verfahren nach Anspruch 2, **dadurch gekennzeichnet, dass** die Punktmutation eine Mutation an der Position 17, 81, 231 oder 251, definiert durch die Position in SEQ ID NO: 3, entstehen lässt.

15. Verfahren nach Anspruch 1, **dadurch gekennzeichnet, dass** die genetische Variation eine Punktmutation des AANAT-Gens ist.

16. Verfahren nach Anspruch 15, **dadurch gekennzeichnet, dass** die Punktmutation eine Mutation an der Position 3, 13, 62, 157 oder 163, definiert durch die Position in SEQ ID NO: 4, entstehen lässt.

17. Verfahren nach Anspruch 16, **dadurch gekennzeichnet, dass** die Mutation in SEQ ID NO: 4 eine T3M-Mutation ist.

18. Verfahren nach Anspruch 16, **dadurch gekennzeichnet, dass** die Mutation in SEQ ID NO: 4 eine A13S-Mutation ist.

19. Verfahren nach Anspruch 16, **dadurch gekennzeichnet, dass** die Mutation in SEQ ID NO: 4 eine V62I-Mutation ist.

20. Verfahren nach Anspruch 16, **dadurch gekennzeichnet, dass** die Mutation in SEQ ID NO: 4 eine A157V-Mutation ist.

21. Verfahren nach Anspruch 16, **dadurch gekennzeichnet, dass** die Mutation in SEQ ID NO: 4 eine A163V-Mutation ist.

## Revendications

1. Procédé de détermination de la probabilité du développement individuel d'un trouble psychiatrique choisi dans le groupe des troubles du spectre autistique (TSA), des troubles des déficits de l'attention et de l'hyperactivité (TDAH) ; comprenant :
- l'identification d'au moins une variation génétique dans un gène choisi dans le groupe constitué des gènes ASMT et AANAT, impliqués dans la modulation de la mélatonine et/ou
- l'analyse de l'activité enzymatique d'une enzyme codée par ledit gène ASMT impliqué dans la voie de la biosynthèse de la mélatonine dans un échantillon dudit individu,
ledit procédé pouvant également comprendre l'étape consistant à :
- analyser le niveau de mélatonine
moyennant quoi l'identification de ladite au moins une variation génétique et/ou diminution de ladite activité enzymatique par rapport à un individu sans trouble psychiatrique, est indicative d'une probabilité que ledit individu développe un trouble psychiatrique.

2. Procédé selon la revendication 1, **caractérisé en ce que** ladite variation génétique est une mutation ponctuelle dans ledit gène ASMT.

3. Procédé selon la revendication 2, **caractérisé en ce que** ladite mutation ponctuelle donne lieu à une mutation en position 17, 81, 210, 306 ou 326 comme défini par la position dans la SEQ. ID N°1.

4. Procédé selon la revendication 3, **caractérisé en ce que** ladite mutation dans la SEQ. ID N°1 est une mutation N17K.

5. Procédé selon la revendication 3, **caractérisé en ce que** ladite mutation dans la SEQ. ID N°1 est une mutation K81E.

6. Procédé selon la revendication 3, **caractérisé en ce que** ladite mutation dans la SEQ. ID N°1 est une mutation R210H.

7. Procédé selon la revendication 3, **caractérisé en ce que** ladite mutation dans la SEQ. ID N°1 est une mutation G306A.

8. Procédé selon la revendication 3, **caractérisé en ce que** ladite mutation dans la SEQ. ID N°1 est une mutation L326F.

9. Procédé selon la revendication 1, **caractérisé en ce que** ladite variation génétique est une mutation du site d'épissage dudit gène ASMT.

10. Procédé selon la revendication 9, **caractérisé en ce que** la mutation du site d'épissage est IVS5+2T>C.

11. Procédé selon la revendication 1, **caractérisé en ce que** ladite variation génétique est située dans un polymorphisme de nucléotides simples (PNS) du gène ASMT.

12. Procédé selon la revendication 11, **caractérisé en ce que** ledit PNS est le Rs5989681 ou le Rs6588809.

13. Procédé selon la revendication 2, **caractérisé en ce que** ladite mutation ponctuelle donne lieu à une mutation en position 17, 81 ou 298, telle que définie par la position dans la SEQ. ID N°2.

14. Procédé selon la revendication 2, **caractérisé en ce que** ladite mutation ponctuelle donne lieu à une mutation en position 17, 81, 231 ou 251, comme défini par la position dans la SEQ. ID N°3.

15. Procédé selon la revendication 1, **caractérisé en ce que** ladite variation génétique est une mutation ponctuelle dudit gène AANAT.

16. Procédé selon la revendication 15, **caractérisé en ce que** ladite mutation ponctuelle donne lieu à une mutation en position 3, 13, 62, 157 ou 163 comme défini par la position dans la SEQ. ID N°4.

17. Procédé selon la revendication 16, **caractérisé en ce que** ladite mutation dans la SEQ. ID N°4 est une mutation T3M.

18. Procédé selon la revendication 16, **caractérisé en ce que** ladite mutation dans la SEQ. ID N°4 est une mutation A13S.

19. Procédé selon la revendication 16, **caractérisé en ce que** ladite mutation dans la SEQ. ID N°4 est une mutation V62I.

20. Procédé selon la revendication 16, **caractérisé en ce que** ladite mutation dans la SEQ. ID N°4 est une mutation A157V.

21. Procédé selon la revendication 16, **caractérisé en ce que** ladite mutation dans la SEQ. ID N°4 est une mutation A163V.
